# FASCICULE DE BREVET EUROPEEN

(11) **EP 1 678 204 B1**
(45) Date de publication et mention de la délivrance du brevet: **20.01.2010**
(21) Numéro de dépôt: 04805350.8
(22) Date de dépôt: 29.10.2004
(51) Int. Cl.: C07K 14/47

(54) **NOUVELLE PROTEINE DE FIXATION DU PHOSPHATE, COMPOSITIONS PHARMACEUTIQUES LA CONTENANT ET SES UTILISATIONS**
NEUES PHOSPHATBINDENDES PROTEIN, PHARMAZEUTISCHE ZUSAMMENSETZUNGEN, DIE DIESES ENTHALTEN, UND VERWENDUNG DAVON
NOVEL PHOSPHATE-BINDING PROTEIN, PHARMACEUTICAL COMPOSITIONS CONTAINING SAME AND USE THEREOF

(30) Priorité: 30.10.2003 FR 0312729
(43) Date de publication de la demande: 12.07.2006
(73) Titulaire: CENTRE NATIONAL DE LA RECHERCHE SCIENTIFIQUE, 75794 Paris Cedex 16 (FR); Université Henri Poincaré (Nancy I), 54013 Nancy Cédex (FR)
(72) Inventeur: CHABRIERE, Eric, F-54000 Nancy (FR); CONTRERAS-MARTEL, Carlos, F-38120 Saint Egreve (FR); FONTECILLA-CAMPS, Juan, 38920 Crolles (FR)
(74) Mandataire: Grosset-Fournier, Chantal Catherine
(86) Numéro de dépôt international: PCT/FR2004/002797
(87) Numéro de publication internationale: WO 2005/042572

(56) Documents cités:
- US-A1- 2003 158 115
- DATABASE UNIPROT [Online] EBI; 10 octobre 2003 (2003-10-10), XP002275669 Database accession no. P35482
- KAWASAKI K. ET AL.: "Mineralized tissue and vertebrate evolution: The secretory calcium-binding phosphoprotein gene cluster" P.N.A.S, vol. 100, no. 7, 1 avril 2003 (2003-04-01), pages 4060-4065, XP002275668

### LISTE DE SEQUENCES

<110> CENTRE NATIONAL DE LA RECHERCHE SCIENTIFIQUE UNIVERSITE HENRI POINCARE DE NANCY
<120> NOUVELLE PROTEINE DE LIAISON AU PHOSPHATE, COMPOSITIONS PHARMACEUTIQUES LA CONTENANT ET SES UTILISATIONS
<130> WOB 03 BU CNR HPBP
<150> FR 03/12729
   <151> 2004-10-30
<160> 11
<170> PatentIn version 3.1
<210> 1
   <211> 376
   <212> PRT
   <213> Homo sapiens
<220>
   <221> MISC_FEATURE
   <222> (1)..(1)
   <223> D ou S
<220>
   <221> MISC_FEATURE
   <222> (3)..(3)
   <223> N ou D
<220>
   <221> MISC_FEATURE
   <222> (11)..(11)
   <223> Q ou E
<220>
   <221> MISC_FEATURE
   <222> (30)..(30)
   <223> V ou T
<220>
   <221> MISC_FEATURE
   <222> (43)..(43)
   <223> K ou S
<220>
   <221> MISC_FEATURE
   <222> (50)..(50)
   <223> D ou N
<220>
   <221> MISC_FEATURE
   <222> (54)..(54)
   <223> N ou D
<220>
   <221> MISC_FEATURE
   <222> (67)..(67)
   <223> T ou S
<220>
   <221> MISC_FEATURE
   <222> (68)..(68)
   <223> E ou Q
<220>
   <221> MISC_FEATURE
   <222> (75)..(75)
   <223> D ou N
<220>
   <221> MISC_FEATURE
   <222> (77)..(77)
   <223> E ou Q
<220>
   <221> MISC_FEATURE
   <222> (85)..(85)
   <223> Q ou E
<220>
   <221> MISC_FEATURE
   <222> (102)..(102)
   <223> A ou G
<220>
   <221> MISC_FEATURE
   <222> (122)..(122)
   <223> D ou N
<220>
   <221> MISC_FEATURE
   <222> (143)..(143)
   <223> S ou V
<220>
   <221> MISC_FEATURE
   <222> (219)..(219)
   <223> T ou S
<220>
   <221> MISC_FEATURE
   <222> (224)..(224)
   <223> D ou N
<220>
   <221> MISC_FEATURE
   <222> (252)..(252)
   <223> V ou S
<220>
   <221> MISC_FEATURE
   <222> (266)..(266)
   <223> D ou N
<400> 1
   Xaa Ile Xaa Gly Gly Gly Ala Thr Leu Pro Xaa Lys Leu Tyr Leu Thr
   1               5                   10                  15
   Pro Asp Val Leu Thr Ala Gly Phe Ala Pro Tyr Ile Gly Xaa Gly Ser
               20                  25                  30
   Gly Lys Gly Lys Ile Ala Phe Leu Glu Asn Xaa Tyr Asn Gln Phe Gly
           35                  40                  45
   Thr Xaa Thr Thr Lys Xaa Val His Trp Ala Gly Ser Asp Ser Lys Leu
       50                  55                  60
   Thr Ala Xaa Xaa Leu Ala Thr Tyr Ala Ala Xaa Lys Xaa Pro Gly Trp
   65                  70                  75                  80
   Gly Lys Leu Ile Xaa Val Pro Ser Val Ala Thr Ser Val Ala Ile Pro
                   85                  90                  95
   Phe Arg Lys Ala Gly Xaa Asn Ala Val Asp Leu Ser Val Lys Glu Leu
               100                 105                 110
   Cys Gly Val Phe Ser Gly Arg Ile Ala Xaa Trp Ser Gly Ile Thr Gly
           115                 120                 125
   Ala Gly Arg Ser Gly Pro Ile Gln Val Val Tyr Arg Ala Glu Xaa Ser
       130                 135                 140
   Gly Thr Thr Glu Leu Phe Thr Arg Phe Leu Asn Ala Lys Cys Thr Thr
   145                 150                 155                 160
   Gln Pro Gly Thr Phe Ala Val Thr Thr Val Phe Ala Asn Ser Tyr Ser
                   165                 170                 175
   Leu Gly Leu Ser Pro Leu Ala Gly Ala Val Ala Ala Ile Gly Ser Val
               180                 185                 190
   Gly Val Met Ala Ala Asp Asn Asp Val Thr Thr Ala Gln Gly Arg Ile
           195                 200                 205
   Thr Tyr Ile Ser Pro Asp Phe Ala Ala Pro Xaa Leu Ala Gly Leu Xaa
       210                 215                 220
   Asp Ala Thr Lys Val Ala Arg Thr Gly Lys Gly Ser Ser Ser Gly Gly
   225                 230                 235                 240
   Gly Ala Glu Gly Lys Ser Pro Ala Ala Ala Asn Xaa Ser Ala Ala Ile
                   245                 250                 255
   Ser Val Val Pro Leu Pro Ala Ala Ala Xaa Arg Gly Asp Pro Asn Val
               260                 265                 270
   Trp Thr Pro Val Phe Gly Ala Val Thr Gly Gly Gly Val Val Ala Tyr
           275                 280                 285
   Pro Asp Ser Gly Tyr Pro Ile Leu Gly Phe Thr Asp Leu Ile Phe Ser
       290                 295                 300
   Glu Cys Tyr Ala Asn Ala Thr Gln Thr Gly Gln Val Arg Asn Phe Phe
   305                 310                 315                 320
   Thr Lys His Tyr Gly Thr Ser Ala Asn Asp Asn Ala Ala Ile Gln Ala
                   325                 330                 335
   Asn Ala Phe Val Pro Leu Pro Ser Asn Trp Lys Ala Ala Val Arg Ala
   340 345 350
   Ser Tyr Leu Thr Ala Ser Asn Ala Leu Ser Ile Gly Asp Ser Ala Val
   355 360 365
   Cys Gly Gly Lys Gly Arg Pro Glu
   370 375
<210> 2
   <211> 376
   <212> PRT
   <213> Homo sapiens
<400> 2
   Asp Ile Asn Gly Gly Gly Ala Thr Leu Pro Gln Lys Leu Tyr Leu Thr
   1               5                   10                  15
   Pro Asp Val Leu Thr Ala Gly Phe Ala Pro Tyr Ile Gly Val Gly Ser
               20                  25                  30
   Gly Lys Gly Lys Ile Ala Phe Leu Glu Asn Lys Tyr Asn Gln Phe Gly
           35                  40                  45
   Thr Asp Thr Thr Lys Asn Val His Trp Ala Gly Ser Asp Ser Lys Leu
       50                  55                  60
   Thr Ala Thr Glu Leu Ala Thr Tyr Ala Ala Asp Lys Glu Pro Gly Trp
   65                  70                  75                  80
   Gly Lys Leu Ile Gln Val Pro Ser Val Ala Thr Ser Val Ala Ile Pro
                   85                  90                  95
   Phe Arg Lys Ala Gly Ala Asn Ala Val Asp Leu Ser Val Lys Glu Leu
               100                 105                 110
   Cys Gly Val Phe Ser Gly Arg Ile Ala Asp Trp Ser Gly Ile Thr Gly
           115                 120                 125
   Ala Gly Arg Ser Gly Pro Ile Gln Val Val Tyr Arg Ala Glu Ser Ser
       130                 135                 140
   Gly Thr Thr Glu Leu Phe Thr Arg Phe Leu Asn Ala Lys Cys Thr Thr
   145                 150                 155                 160
   Gln Pro Gly Thr Phe Ala Val Thr Thr Val Phe Ala Asn Ser Tyr Ser
                   165                 170                 175
   Leu Gly Leu Ser Pro Leu Ala Gly Ala Val Ala Ala Ile Gly Ser Val
               180                 185                 190
   Gly Val Met Ala Ala Asp Asn Asp Val Thr Thr Ala Gln Gly Arg Ile
           195                 200                 205
   Thr Tyr Ile Ser Pro Asp Phe Ala Ala Pro Thr Leu Ala Gly Leu Asp
       210                 215                 220
   Asp Ala Thr Lys Val Ala Arg Thr Gly Lys Gly Ser Ser Ser Gly Gly
   225                 230                 235                 240
   Gly Ala Glu Gly Lys Ser Pro Ala Ala Ala Asn Val Ser Ala Ala Ile
                   245                 250                 255
   Ser Val Val Pro Leu Pro Ala Ala Ala Asp Arg Gly Asp Pro Asn Val
               260                 265                 270
   Trp Thr Pro Val Phe Gly Ala Val Thr Gly Gly Gly Val Val Ala Tyr
           275                 280                 285
   Pro Asp Ser Gly Tyr Pro Ile Leu Gly Phe Thr Asp Leu Ile Phe Ser
       290                 295                 300
   Glu Cys Tyr Ala Asn Ala Thr Gln Thr Gly Gln Val Arg Asn Phe Phe
   305                 310                 315                 320
   Thr Lys His Tyr Gly Thr Ser Ala Asn Asp Asn Ala Ala Ile Gln Ala
                   325                 330                 335
   Asn Ala Phe Val Pro Leu Pro Ser Asn Trp Lys Ala Ala Val Arg Ala
               340                 345                 350
   Ser Tyr Leu Thr Ala Ser Asn Ala Leu Ser Ile Gly Asp Ser Ala Val
           355                 360                 365
   Cys Gly Gly Lys Gly Arg Pro Glu
       370                 375
<210> 3
   <211> 376
   <212> PRT
   <213> Homo sapiens
<400> 3
   Ser Ile Asp Gly Gly Gly Ala Thr Leu Pro Glu Lys Leu Tyr Leu Thr
   1               5                   10                  15
   Pro Asp Val Leu Thr Ala Gly Phe Ala Pro Tyr Ile Gly Thr Gly Ser
               20                  25                  30
   Gly Lys Gly Lys Ile Ala Phe Leu Glu Asn Ser Tyr Asn Gln Phe Gly
           35                  40                  45
   Thr Asn Thr Thr Lys Asp Val His Trp Ala Gly Ser Asp Ser Lys Leu
       50                  55                  60
   Thr Ala Ser Gln Leu Ala Thr Tyr Ala Ala Asn Lys Gln Pro Gly Trp
   65                  70                  75                  80
   Gly Lys Leu Ile Glu Val Pro Ser Val Ala Thr Ser Val Ala Ile Pro
                   85                  90                  95
   Phe Arg Lys Ala Gly Gly Asn Ala Val Asp Leu Ser Val Lys Glu Leu
               100                 105                 110
   Cys Gly Val Phe Ser Gly Arg Ile Ala Asn Trp Ser Gly Ile Thr Gly
           115                 120                 125
   Ala Gly Arg Ser Gly Pro Ile Gln Val Val Tyr Arg Ala Glu Val Ser
       130                 135                 140
   Gly Thr Thr Glu Leu Phe Thr Arg Phe Leu Asn Ala Lys Cys Thr Thr
   145                 150                 155                 160
   Gln Pro Gly Thr Phe Ala Val Thr Thr Val Phe Ala Asn Ser Tyr Ser
                   165                 170                 175
   Leu Gly Leu Ser Pro Leu Ala Gly Ala Val Ala Ala Ile Gly Ser Val
               180                 185                 190
   Gly Val Met Ala Ala Asp Asn Asp Val Thr Thr Ala Gln Gly Arg Ile
           195                 200                 205
   Thr Tyr Ile Ser Pro Asp Phe Ala Ala Pro Ser Leu Ala Gly Leu Asn
       210                 215                 220
   Asp Ala Thr Lys Val Ala Arg Thr Gly Lys Gly Ser Ser Ser Gly Gly
   225                 230                 235                 240
   Gly Ala Glu Gly Lys Ser Pro Ala Ala Ala Asn Ser Ser Ala Ala Ile
                   245                 250                 255
   Ser Val Val Pro Leu Pro Ala Ala Ala Asn Arg Gly Asp Pro Asn Val
               260                 265                 270
   Trp Thr Pro Val Phe Gly Ala Val Thr Gly Gly Gly Val Val Ala Tyr
           275                 280                 285
   Pro Asp Ser Gly Tyr Pro Ile Leu Gly Phe Thr Asp Leu Ile Phe Ser
       290                 295                 300
   Glu Cys Tyr Ala Asn Ala Thr Gln Thr Gly Gln Val Arg Asn Phe Phe
   305                 310                 315                 320
   Thr Lys His Tyr Gly Thr Ser Ala Asn Asp Asn Ala Ala Ile Gln Ala
                   325                 330                 335
   Asn Ala Phe Val Pro Leu Pro Ser Asn Trp Lys Ala Ala Val Arg Ala
               340                 345                 350
   Ser Tyr Leu Thr Ala Ser Asn Ala Leu Ser Ile Gly Asp Ser Ala Val
           355                 360                 365
   Cys Gly Gly Lys Gly Arg Pro Glu
       370                 375
<210> 4
   <211> 355
   <212> PRT
   <213> Homo sapiens
<400> 4
   Met Ala Lys Leu Ile Ala Leu Thr Leu Leu Gly Met Gly Leu Ala Leu
   1               5                   10                  15
   Phe Arg Asn His Gln Ser Ser Tyr Gln Thr Arg Leu Asn Ala Leu Arg
               20                  25                  30
   Glu Val Gln Pro Val Glu Leu Pro Asn Cys Asn Leu Val Lys Gly Ile
           35                  40                  45
   Glu Thr Gly Ser Glu Asp Met Glu Ile Leu Pro Asn Gly Leu Ala Phe
       50                  55                  60
   Ile Ser Ser Gly Leu Lys Tyr Pro Gly Ile Lys Ser Phe Asn Pro Asn
   65                  70                  75                  80
   Ser Pro Gly Lys Ile Leu Leu Met Asp Leu Asn Glu Glu Asp Pro Thr
                   85                  90                  95
   Val Leu Glu Leu Gly Ile Thr Gly Ser Lys Phe Asp Val Ser Ser Phe
               100                 105                 110
   Asn Pro His Gly Ile Ser Thr Phe Thr Asp Glu Asp Asn Ala Met Tyr
           115                 120                 125
   Leu Leu Val Val Asn His Pro Asp Ala Lys Ser Thr Val Glu Leu Phe
       130                 135                 140
   Lys Phe Gln Glu Glu Glu Lys Ser Leu Leu His Leu Lys Thr Ile Arg
   145                 150                 155                 160
   His Lys Leu Leu Pro Asn Leu Asn Asp Ile Val Ala Val Gly Pro Glu
                   165                 170                 175
   His Phe Tyr Gly Thr Asn Asp His Tyr Phe Leu Asp Pro Tyr Leu Gln
               180                 185                 190
   Ser Trp Glu Met Tyr Leu Gly Leu Ala Trp Ser Tyr Val Val Tyr Tyr
           195                 200                 205
   Ser Pro Ser Glu Val Arg Val Val Ala Glu Gly Phe Asp Phe Ala Asn
       210                 215                 220
   Gly Ile Asn Ile Ser Pro Asp Gly Lys Tyr Val Tyr Ile Ala Glu Leu
   225                 230                 235                 240
   Leu Ala His Lys Ile His Val Tyr Glu Lys His Ala Asn Trp Thr Leu
                   245                 250                 255
   Thr Pro Leu Lys Ser Leu Asp Phe Asn Thr Leu Val Asp Asn Ile Ser
               260                 265                 270
   Val Asp Pro Glu Thr Gly Asp Leu Trp Val Gly Cys His Pro Asn Gly
           275                 280                 285
   Met Lys Ile Phe Phe Tyr Asp Ser Glu Asn Pro Pro Ala Ser Glu Val
       290                 295                 300
   Leu Arg Ile Gln Asn Ile Leu Thr Glu Glu Pro Lys Val Thr Gln Val
   305                 310                 315                 320
   Tyr Ala Glu Asn Gly Thr Val Leu Gln Gly Ser Thr Val Ala Ser Val
                   325                 330                 335
   Tyr Lys Gly Lys Leu Leu Ile Gly Thr Val Phe His Lys Ala Leu Tyr
               340                 345                 350
   Cys Glu Leu
           355
<210> 5
   <211> 354
   <212> PRT
   <213> Homo sapiens
<400> 5
   Met Gly Ala Trp Val Gly Cys Gly Leu Ala Gly Asp Arg Ala Gly Phe
   1               5                   10                  15
   Leu Gly Glu Arg Leu Leu Ala Leu Arg Asn Arg Leu Lys Ala Ser Arg
               20                  25                  30
   Glu Val Glu Ser Val Asp Leu Pro His Cys His Leu Ile Lys Gly Ile
           35                  40                  45
   Glu Ala Gly Ser Glu Asp Ile Asp Ile Leu Pro Asn Gly Leu Ala Phe
       50                  55                  60
   Phe Ser Val Gly Leu Lys Phe Pro Gly Leu His Ser Phe Ala Pro Asp
   65                  70                  75                  80
   Lys Pro Gly Gly Ile Leu Met Met Asp Leu Lys Glu Glu Lys Pro Arg
                   85                  90                  95
   Ala Arg Glu Leu Arg Ile Ser Arg Gly Phe Asp Leu Ala Ser Phe Asn
               100                 105                 110
   Pro His Gly Ile Ser Thr Phe Ile Asp Asn Asp Asp Thr Val Tyr Leu
           115                 120                 125
   Phe Val Val Asn His Pro Glu Phe Lys Asn Thr Val Glu Ile Phe Lys
       130                 135                 140
   Phe Glu Glu Ala Glu Asn Ser Leu Leu His Leu Lys Thr Val Lys His
   145                 150                 155                 160
   Glu Leu Leu Pro Ser Val Asn Asp Ile Thr Ala Val Gly Pro Ala His
                   165                 170                 175
   Phe Tyr Ala Thr Asn Asp His Tyr Phe Ser Asp Pro Phe Leu Lys Tyr
               180                 185                 190
   Leu Glu Thr Tyr Leu Asn Leu His Trp Ala Asn Val Val Tyr Tyr Ser
           195                 200                 205
   Pro Asn Glu Val Lys Val Val Ala Glu Gly Phe Asp Ser Ala Asn Gly
       210                 215                 220
   Ile Asn Ile Ser Pro Asp Asp Lys Tyr Ile Tyr Val Ala Asp Ile Leu
   225                 230                 235                 240
   Ala His Glu Ile His Val Leu Glu Lys His Thr Asn Met Asn Leu Thr
                   245                 250                 255
   Gln Leu Lys Val Leu Glu Leu Asp Thr Leu Val Asp Asn Leu Ser Ile
               260                 265                 270
   Asp Pro Ser Ser Gly Asp Ile Trp Val Gly Cys His Pro Asn Gly Gln
           275                 280                 285
   Lys Leu Phe Val Tyr Asp Pro Asn Asn Pro Pro Ser Ser Glu Val Leu
       290                 295                 300
   Arg Ile Gln Asn Ile Leu Cys Glu Lys Pro Thr Val Thr Thr Val Tyr
   305                 310                 315                 320
   Ala Asn Asn Gly Ser Val Leu Gln Gly Ser Ser Val Ala Ser Val Tyr
                   325                 330                 335
   Asp Gly Lys Leu Leu Ile Gly Thr Leu Tyr His Arg Ala Leu Tyr Cys
               340                 345                 350
   Glu Leu
<210> 6
   <211> 354
   <212> PRT
   <213> Homo sapiens
<400> 6
   Met Gly Lys Leu Val Ala Leu Val Leu Leu Gly Val Gly Leu Ser Leu
   1               5                   10                  15
   Val Gly Glu Met Phe Leu Ala Phe Arg Glu Arg Val Asn Ala Ser Arg
               20                  25                  30
   Glu Val Glu Pro Val Glu Pro Glu Asn Cys His Leu Ile Glu Glu Leu
           35                  40                  45
   Glu Ser Gly Ser Glu Asp Ile Asp Ile Leu Pro Ser Gly Leu Ala Phe
       50                  55                  60
   Ile Ser Ser Gly Leu Lys Tyr Pro Gly Met Pro Asn Phe Ala Pro Asp
   65                  70                  75                  80
   Glu Pro Gly Lys Ile Phe Leu Met Asp Leu Asn Glu Gln Asn Pro Arg
                   85                  90                  95
   Ala Gln Ala Leu Glu Ile Ser Gly Gly Phe Asp Lys Glu Leu Phe Asn
               100                 105                 110
   Pro His Gly Ile Ser Ile Phe Ile Asp Lys Asp Asn Thr Val Tyr Leu
           115                 120                 125
   Tyr Val Val Asn His Pro His Met Lys Ser Thr Val Glu Ile Phe Lys
       130                 135                 140
   Phe Glu Glu Gln Gln Arg Ser Leu Val Tyr Leu Lys Thr Ile Lys His
   145                 150                 155                 160
   Glu Leu Leu Lys Ser Val Asn Asp Ile Val Val Leu Gly Pro Glu Gln
                   165                 170                 175
   Phe Tyr Ala Thr Arg Asp His Tyr Phe Thr Asn Ser Leu Leu Ser Phe
               180                 185                 190
   Phe Glu Met Ile Leu Asp Leu Arg Trp Thr Tyr Val Leu Phe Tyr Ser
           195                 200                 205
   Pro Arg Glu Val Lys Val Val Ala Lys Gly Phe Cys Ser Ala Asn Gly
       210                 215                 220
   Ile Thr Val Ser Ala Asp Gln Lys Tyr Val Tyr Val Ala Asp Val Ala
   225                 230                 235                 240
   Ala Lys Asn Ile His Ile Met Glu Lys His Asp Asn Trp Asp Leu Thr
                   245                 250                 255
   Gln Leu Lys Val Ile Gln Leu Gly Thr Leu Val Asp Asn Leu Thr Val
               260                 265                 270
   Asp Pro Ala Thr Gly Asp Ile Leu Ala Gly Cys His Pro Asn Pro Met
           275                 280                 285
   Lys Leu Leu Asn Tyr Asn Pro Glu Asp Pro Pro Gly Ser Glu Val Leu
       290                 295                 300
   Arg Ile Gln Asn Val Leu Ser Glu Lys Pro Arg Val Ser Thr Val Tyr
   305                 310                 315                 320
   Ala Asn Asn Gly Ser Val Leu Gln Gly Thr Ser Val Ala Ser Val Tyr
                   325                 330                 335
   His Gly Lys Ile Leu Ile Gly Thr Val Phe His Lys Thr Leu Tyr Cys
               340                 345                 350
   Glu Leu
<210> 7
   <211> 359
   <212> PRT
   <213> Oryctolagus cuniculus
<400> 7
   Met Ala Lys Leu Thr Ala Leu Thr Leu Leu Gly Leu Gly Leu Ala Leu
   1               5                   10                  15
   Phe Asp Gly Gln Lys Ser Ser Phe Gln Thr Arg Phe Asn Val His Arg
               20                  25                  30
   Glu Val Thr Pro Val Glu Leu Pro Asn Cys Asn Leu Val Lys Gly Ile
           35                  40                  45
   Asp Asn Gly Ser Glu Asp Leu Glu Ile Leu Pro Asn Gly Leu Ala Phe
       50                  55                  60
   Ile Ser Ala Gly Leu Lys Tyr Pro Gly Ile Met Ser Phe Asp Pro Asp
   65                  70                  75                  80
   Lys Pro Gly Lys Ile Leu Leu Met Asp Leu Asn Glu Lys Asp Pro Val
                   85                  90                  95
   Val Leu Glu Leu Ser Ile Thr Gly Ser Thr Phe Asp Leu Ser Ser Phe
               100                 105                 110
   Asn Pro His Gly Ile Ser Thr Phe Thr Asp Glu Asp Asn Ile Val Tyr
           115                 120                 125
   Leu Met Val Val Asn His Pro Asp Ser Lys Ser Thr Val Glu Leu Phe
       130                 135                 140
   Lys Phe Gln Glu Lys Glu Lys Ser Leu Leu His Leu Lys Thr Ile Arg
   145                 150                 155                 160
   His Lys Leu Leu Pro Ser Val Asn Asp Ile Val Ala Val Gly Pro Glu
                   165                 170                 175
   His Phe Tyr Ala Thr Asn Asp His Tyr Phe Ile Asp Pro Tyr Leu Lys
               180                 185                 190
   Ser Trp Glu Met His Leu Gly Leu Ala Trp Ser Phe Val Thr Tyr Tyr
           195                 200                 205
   Ser Pro Asn Asp Val Arg Val Val Ala Glu Gly Phe Asp Phe Ala Asn
       210                 215                 220
   Gly Ile Asn Ile Ser Pro Asp Gly Lys Tyr Val Tyr Ile Ala Glu Leu
   225                 230                 235                 240
   Leu Ala His Lys Ile His Val Tyr Glu Lys His Ala Asn Trp Thr Leu
                   245                 250                 255
   Thr Pro Leu Lys Ser Leu Asp Phe Asn Thr Leu Val Asp Asn Ile Ser
               260                 265                 270
   Val Asp Pro Val Thr Gly Asp Leu Trp Val Gly Cys His Pro Asn Gly
           275                 280                 285
   Met Arg Ile Phe Tyr Tyr Asp Pro Lys Asn Pro Pro Ala Ser Glu Val
       290                 295                 300
   Leu Arg Ile Gln Asp Ile Leu Ser Lys Glu Pro Lys Val Thr Val Ala
   305                 310                 315                 320
   Tyr Ala Glu Asn Gly Thr Val Leu Gln Gly Ser Thr Val Ala Ala Val
                   325                 330                 335
   Tyr Lys Gly Lys Met Leu Val Gly Thr Val Phe His Lys Ala Leu Tyr
               340                 345                 350
   Cys Glu Leu Ser Gln Ala Asn
           355
<210> 8
   <211> 355
   <212> PRT
   <213> Rattus rattus
<400> 8
   Met Ala Lys Leu Leu Gly Leu Thr Leu Val Gly Leu Val Leu Ala Leu
   1               5                   10                  15
   Tyr Lys Asn His Arg Ser Ser Tyr Gln Thr Arg Leu Asn Ala Phe Arg
               20                  25                  30
   Glu Val Thr Pro Val Asp Leu Pro Asn Cys Thr Leu Val Lys Gly Ile
           35                  40                  45
   Glu Ala Gly Ala Glu Asp Leu Glu Ile Leu Pro Asn Gly Leu Thr Phe
       50                  55                  60
   Phe Ser Thr Phe Leu Lys Tyr Pro Gly Ile Lys Ser Phe Asp Pro Ser
   65                  70                  75                  80
   Lys Pro Gly Lys Ile Leu Leu Met Asp Leu Asn Glu Lys Glu Pro Ala
                   85                  90                  95
   Val Ser Glu Leu Ala Ile Met Gly Asn Thr Leu Asp Met Ser Ser Phe
               100                 105                 110
   Asn Pro His Gly Ile Ser Thr Phe Ile Asp Glu Asp Asn Thr Val Tyr
           115                 120                 125
   Leu Leu Val Val Ser His Pro Asp Ser Ser Ser Thr Val Glu Val Phe
       130                 135                 140
   Lys Phe Gln Glu Glu Glu Arg Ser Leu Leu His Leu Lys Thr Ile Thr
   145                 150                 155                 160
   His Glu Leu Leu Pro Ser Ile Asn Asp Ile Ala Ala Val Gly Pro Glu
                   165                 170                 175
   Ser Phe Tyr Ala Thr Asn Asp His Tyr Phe Ala Asp Pro Tyr Leu Arg
               180                 185                 190
   Ser Trp Glu Met Tyr Leu Gly Leu Ser Trp Ser Asn Val Val Tyr Tyr
           195                 200                 205
   Ser Pro Asp Lys Val Arg Val Val Ala Asp Gly Phe Asp Phe Ala Asn
       210                 215                 220
   Gly Ile Gly Ile Ser Leu Asp Gly Lys Tyr Val Tyr Ile Ala Glu Leu
   225                 230                 235                 240
   Leu Ala His Lys Ile His Val Tyr Glu Lys His Ala Asn Trp Thr Leu
                   245                 250                 255
   Thr Pro Leu Lys Val Leu Ser Phe Asp Thr Leu Val Asp Asn Ile Ser
               260                 265                 270
   Val Asp Pro Val Thr Gly Asp Leu Trp Val Gly Cys His Pro Asn Gly
           275                 280                 285
   Met Arg Ile Phe Phe Tyr Asp Ser Glu Asn Pro Pro Gly Ser Glu Val
       290                 295                 300
   Leu Arg Ile Gln Ser Ile Leu Ser Glu Asp Pro Lys Val Thr Val Val
   305                 310                 315                 320
   Tyr Ala Glu Asn Gly Thr Val Leu Gln Gly Thr Thr Val Ala Ala Val
                   325                 330                 335
   Tyr Lys Gly Lys Leu Leu Ile Gly Thr Val Phe His Arg Ala Leu Cys
               340                 345                 350
   Cys Tyr Leu
           355
<210> 9
   <211> 355
   <212> PRT
   <213> Mus musculus
<400> 9
   Met Ala Lys Leu Leu Ala Leu Thr Leu Val Gly Leu Val Leu Ala Leu
   1               5                   10                  15
   Tyr Lys Asn His Arg Ser Ser Tyr Gln Thr Arg Leu Asn Ala Phe Arg
               20                  25                  30
   Glu Val Thr Pro Val Glu Leu Pro Asn Cys Asn Leu Val Lys Gly Ile
           35                  40                  45
   Glu Thr Gly Ala Glu Asp Leu Glu Ile Leu Pro Asn Gly Leu Thr Phe
       50                  55                  60
   Phe Ser Thr Gly Leu Lys Tyr Pro Gly Ile Lys Ser Phe Asp Pro Ser
   65                  70                  75                  80
   Lys Pro Gly Lys Ile Leu Leu Met Asp Leu Asn Lys Lys Glu Pro Ala
                   85                  90                  95
   Val Ser Glu Leu Glu Ile Ile Gly Asn Thr Leu Asp Ile Ser Ser Phe
               100                 105                 110
   Asn Pro His Gly Ile Ser Thr Phe Thr Asp Glu Asp Asn Thr Val Tyr
           115                 120                 125
   Leu Leu Val Val Asn His Pro Asp Ser Ser Ser Thr Val Glu Val Phe
       130                 135                 140
   Lys Phe Gln Glu Glu Glu Arg Ser Leu Leu His Leu Lys Thr Ile Thr
   145                 150                 155                 160
   His Glu Leu Leu Pro Ser Ile Asn Asp Ile Ala Ala Ile Gly Pro Glu
                   165                 170                 175
   Ser Phe Tyr Ala Thr Asn Asp His Tyr Phe Ala Asp Pro Tyr Leu Arg
               180                 185                 190
   Ser Trp Glu Met Tyr Leu Gly Leu Ser Trp Ser Asn Val Val Tyr Tyr
           195                 200                 205
   Ser Pro Asp Lys Val Gln Val Val Ala Glu Gly Phe Asp Phe Ala Asn
       210                 215                 220
   Gly Ile Gly Ile Ser Leu Asp Gly Lys Tyr Val Tyr Ile Ala Glu Leu
   225                 230                 235                 240
   Leu Ala His Lys Ile His Val Tyr Glu Lys His Ala Asn Trp Thr Leu
                   245                 250                 255
   Thr Pro Leu Lys Val Leu Asn Phe Asp Thr Leu Val Asp Asn Ile Ser
               260                 265                 270
   Val Asp Pro Val Thr Gly Asp Leu Trp Val Gly Cys His Pro Asn Gly
           275                 280                 285
   Met Arg Ile Phe Phe Tyr Asp Ala Glu Asn Pro Pro Gly Ser Glu Val
   290 295 300
   Leu Arg Ile Gln Asn Ile Leu Ser Glu Asp Pro Lys Ile Thr Val Val
   305 310 315 320
   Tyr Ala Glu Asn Gly Thr Val Leu Gln Gly Thr Thr Val Ala Ser Val
   325 330 335
   Tyr Lys Gly Lys Leu Leu Ile Gly Thr Val Phe His Lys Ala Leu Tyr
   340 345 350
   Cys Asp Leu
   355
<210> 10
   <211> 354
   <212> PRT
   <213> Mus musculus
<400> 10
   Met Gly Arg Met Val Ala Leu Gly Phe Ala Gly His Arg Val Ala Leu
   1               5                   10                  15
   Leu Gly Glu Arg Phe Leu Ala Leu Ser Ser Arg Leu Lys Gly Ser Arg
               20                  25                  30
   Glu Val Glu Ser Val Asp Leu Pro Asn Cys His Leu Ile Lys Gly Ile
           35                  40                  45
   Glu Thr Gly Ala Glu Asp Ile Asp Ile Leu Pro Asn Gly Leu Ala Phe
       50                  55                  60
   Phe Ser Val Gly Leu Lys Phe Pro Gly Leu His Ser Phe Ala Pro Asp
   65                  70                  75                  80
   Lys Pro Gly Gly Ile Leu Met Met Asp Leu Asp Glu Arg Pro Pro Ser
                   85                  90                  95
   Leu Glu Glu Leu Arg Val Ser Trp Gly Phe Asp Leu Ala Ser Phe Asn
               100                 105                 110
   Pro His Gly Ile Ser Thr Phe Ile Asp Asp Asp Asp Thr Val Tyr Leu
           115                 120                 125
   Phe Val Val Asn His Pro Gln Phe Ser Asn Thr Val Glu Ile Phe Lys
       130                 135                 140
   Phe Gln Glu Ala Glu Asn Ser Leu Leu His Leu Lys Thr Ile Lys His
   145                 150                 155                 160
   Glu Leu Leu Pro Ser Val Asn Asp Ile Ile Ala Val Gly Pro Ala His
                   165                 170                 175
   Phe Tyr Ala Thr Asn Asp His Tyr Phe Ser Asp Pro Phe Leu Lys Tyr
               180                 185                 190
   Leu Glu Thr Tyr Leu Asn Leu His Trp Ala Asn Val Val Tyr Tyr Ser
           195                 200                 205
   Pro Glu Glu Val Lys Leu Val Ala Glu Gly Phe Asp Ser Ala Asn Gly
       210                 215                 220
   Ile Asn Ile Ser Pro Asp Lys Lys Tyr Val Tyr Val Ala Asp Ile Leu
   225                 230                 235                 240
   Ala His Glu Ile His Val Leu Glu Lys Gln Pro Asn Met Asn Leu Thr
                   245                 250                 255
   Gln Leu Lys Val Leu Gln Leu Gly Thr Leu Val Asp Asn Leu Ser Ile
               260                 265                 270
   Asp Pro Ser Ser Gly Asp Ile Trp Val Gly Cys His Pro Asn Gly Gln
           275                 280                 285
   Arg Leu Phe Val Tyr His Pro Asn His Pro Pro Thr Ser Glu Val Leu
       290                 295                 300
   Arg Ile Gln Asn Ile Leu Ser Glu Lys Pro Ser Val Thr Thr Val Tyr
   305                 310                 315                 320
   Ile Asn Asn Gly Ser Val Leu Gln Gly Ser Ser Val Ala Thr Ile Tyr
                   325                 330                 335
   Asp Arg Lys Leu Leu Val Gly Thr Leu Tyr Gln Lys Ala Leu Tyr Cys
               340                 345                 350
   Glu Leu
<210> 11
   <211> 354
   <212> PRT
   <213> Mus musculus
<400> 11
   Met Gly Lys Leu Val Ala Leu Thr Leu Leu Gly Ala Cys Leu Ala Leu
   1               5                   10                  15
   Ile Gly Glu Arg Leu Leu Asn Phe Arg Glu Arg Val Ser Thr Thr Arg
               20                  25                  30
   Glu Ile Lys Ala Thr Glu Pro Gln Asn Cys His Leu Ile Glu Gly Leu
           35                  40                  45
   Glu Asn Gly Ser Glu Asp Ile Asp Ile Leu Pro Ser Gly Leu Ala Phe
       50                  55                  60
   Ile Ser Thr Gly Leu Lys Tyr Pro Gly Met Pro Ala Phe Ala Pro Asp
   65                  70                  75                  80
   Lys Pro Gly Arg Ile Phe Leu Met Asp Leu Asn Glu Gln Asn Pro Glu
                   85                  90                  95
   Ala Gln Ala Leu Glu Ile Ser Gly Gly Leu Asp Gln Glu Ser Leu Asn
               100                 105                 110
   Pro His Gly Ile Ser Thr Phe Ile Asp Lys Asp Asn Thr Ala Tyr Leu
           115                 120                 125
   Tyr Val Val Asn His Pro Asn Met Asp Ser Thr Val Glu Ile Phe Lys
       130                 135                 140
   Phe Glu Glu Gln Gln Arg Ser Leu Ile His Leu Lys Thr Leu Lys His
   145                 150                 155                 160
   Glu Leu Leu Lys Ser Val Asn Asp Ile Val Val Leu Gly Pro Glu Gln
                   165                 170                 175
   Phe Tyr Ala Thr Arg Asp His Tyr Phe Thr Ser Tyr Phe Leu Val Leu
               180                 185                 190
   Leu Glu Met Ile Leu Asp Pro His Trp Thr Ser Val Val Phe Tyr Ser
           195                 200                 205
   Pro Lys Glu Val Lys Val Val Ala Gln Gly Phe Ser Ser Ala Asn Gly
       210                 215                 220
   Ile Thr Val Ser Leu Asp Gln Lys Phe Val Tyr Val Ala Asp Val Thr
   225                 230                 235                 240
   Ala Lys Asn Ile His Ile Met Lys Lys His Asp Asn Trp Asp Leu Thr
                   245                 250                 255
   Pro Val Lys Val Ile Gln Leu Gly Thr Leu Val Asp Asn Leu Thr Val
               260                 265                 270
   Asp Pro Ala Thr Gly Asp Ile Leu Ala Gly Cys His Pro Asn Pro Met
           275                 280                 285
   Lys Leu Leu Ile Tyr Asn Pro Glu Asp Pro Pro Gly Ser Glu Val Leu
       290                 295                 300
   Arg Ile Gln Asp Ser Leu Ser Asp Lys Pro Arg Val Ser Thr Leu Tyr
   305                 310                 315                 320
   Ala Asn Asn Gly Ser Val Leu Gln Gly Ser Thr Val Ala Ser Val Tyr
                   325                 330                 335
   His Lys Arg Met Leu Ile Gly Thr Ile Phe His Lys Ala Leu Tyr Cys
               340                 345                 350
   Asp Leu

## Description

La présente invention a pour objet une nouvelle protéine, issue du sérum humain, de fixation du phosphate, des compositions pharmaceutiques la contenant ainsi que ses utilisations, notamment dans le cadre du traitement de l'hyperphosphatémie et des maladies cardiovasculaires ou de l'arthrite.

Le phosphate est une molécule très importante impliquée dans de nombreux mécanismes biologiques. On retrouve notamment le phosphate dans les phospholipides, dans le mécanisme de production d'énergie (ATP, ADP), dans les processus de signalisation cellulaire, dans la composition du matériel génétique dans les os (sous forme de phosphate de calcium).

L'hyperphosphatémie est une pathologie liée à un excès de phosphate dans l'organisme et provoque notamment une augmentation des risques de maladies cardiovasculaires, en favorisant les processus d'athérosclérose et de calcification des artères (Dorozhkin et Epple, 2002 ; Amann et al., 2003 ; Blazheevich et al., 1975). La calcification s'effectuant au niveau des articulations, l'hyperphosphatémie peut aussi provoquer de l'arthrite (pseudo-goutte).

Les sels de phosphate de calcium produits dans le sérum lors d'une hyperphosphatémie précipitent dans les tissus mous avec calcification ectopique dans différents tissus : vaisseaux (accidents vasculaires cérébraux ou cardiaques), articulations (pseudo-goutte), cristallin, interstitium rénal (néphrocalcinose), sous-cutanées (prurit), pulmonaires, pancréatiques.

Ainsi, la moitié des décès chez les personnes souffrant d'insuffisance rénale est due à des maladies cardiovasculaires liées à l'hyperphosphatémie. A cet égard, certains chélateurs du phosphate qui complexent le phosphate dans la lumière intestinale sont actuellement utilisés comme médicament. Cependant, tous ces chélateurs ne sont pas physiologiques. De là découlent certaines complications ou restrictions quant à leur usage.

Les préparations contenant du magnésium sont limitées par la survenue de troubles digestifs (diarrhée) et sont à proscrire en raison du risque d'hypermagnésémie. De même, la prescription d'hydroxyde d'aluminium, longtemps utilisé du fait de son efficacité, doit être évitée, ou du moins limitée à de très faibles périodes, en raison du risque d'intoxication aluminique (anémie hypochrome microcytaire, bstéomalacie, myopathie, démence).

La prescription de sels de calcium est le meilleur moyen pour corriger à la fois l'hypocalcémie et l'hyperphosphorémie, permettant d'une part d'augmenter la quantité de calcium absorbée par l'intestin grêle malgré le déficit en calcitriol, et d'autre part de complexer le phosphore dans la lumière intestinale sous forme de phosphate de calcium qui sera éliminé dans les selles. Cependant, l'inconvénient majeur des chélateurs contenant du calcium est d'induire une hypercalcémie, qui, dans certaines séries, a pu être notée chez 20% des malades. Ce risque a conduit à mettre au point d'autres produits capables de limiter l'hyperphosphorémie.

Le médicament actuellement le plus utilisé est le Renagel^{®} (Ramsdell ; 1999). Il s'agit d'un polymère cationique, non absorbable capable de chélater le phosphate.

La présente invention a pour but de fournir un nouveau chélateur protéique physiologique se liant au phosphate, ne nécessitant pas l'emploi d'autres ions qui peuvent entraîner des complications et offrant de plus larges perspectives d'utilisation que les chélateurs actuels.

La présente invention concerne une protéine caractérisée en ce qu'elle comprend ou est constituée par :
- la séquence SEQ ID NO : 1,
- ou toute séquence dérivée de la séquence SEQ ID NO: 1, notamment par substitution, suppression ou addition d'un ou plusieurs acides aminés, sous réserve que ladite séquence dérivée se lie au phosphate,
- ou toute séquence homologue de la séquence SEQ ID NO: 1, ayant de préférence une homologie d'au moins environ 80% avec la séquence SEQ ID NO : 1, sous réserve que ladite séquence homologue se lie au phosphate,
- ou tout fragment d'une des séquences définies ci-dessus, sous réserve que ledit fragment se lie au phosphate, notamment tout fragment étant constitué d'au moins environ 20 acides aminés contigus dans la séquence SEQ ID NO : 1.

La présente invention concerne une protéine telle que définie ci-dessus, caractérisée en ce qu'elle comprend ou est constituée par :
- la séquence SEQ ID NO : 2 ou la séquence SEQ ID NO : 3,
- ou toute séquence dérivée de la séquence SEQ ID NO : 2 ou SEQ ID NO : 3, notamment par substitution, suppression ou addition d'un ou plusieurs acides aminés, sous réserve que ladite séquence dérivée se lie au phosphate,
- ou toute séquence homologue de la séquence SEQ ID NO: 2 ou SEQ ID NO : 3, ayant de préférence une homologie d'au moins environ 80% avec la séquence SEQ ID NO : 2 ou SEQ ID NO : 3, sous réserve que ladite séquence homologue se lie au phosphate,
- ou tout fragment d'une des séquences définies ci-dessus, sous réserve que ledit fragment se lie au phosphate, notamment tout fragment étant constitué d'au moins environ 20 acides aminés contigus dans la séquence SEQ ID NO : 2 ou SEQ ID NO : 3.

La séquence SEQ ID NO : 2 correspond à la protéine humaine de fixation du phosphate. Cette nouvelle protéine a été isolée dans le plasma humain et sa structure tridimensionnelle montre qu'elle appartient à la classe des "phosphate binding protein" (protéines de fixation du phosphate : PBP). Elle est également appelée par la suite HPBP (protéine humaine de fixation du phosphate).

La séquence SEQ ID NO : 3 correspond à une protéine homologue de la protéine de séquence SEQ ID NO : 2, présentant un pourcentage d'identité d'environ 90% avec la séquence SEQ ID NO : 2, et ayant les mêmes propriétés de fixation du phosphate que la séquence SEQ ID NO : 2.

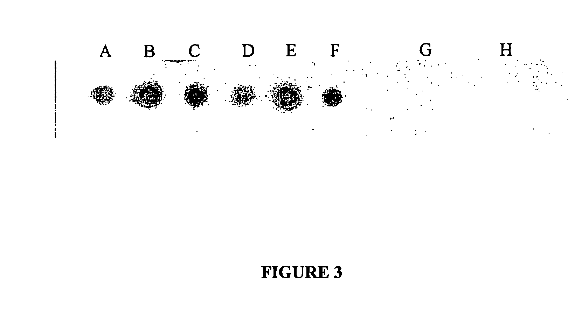

La propriété de fixation du phosphate des séquences de l'invention peut être vérifiée par le test suivant de fixation du phosphate par marquage radioactif :
La protéine est fixée sur une membrane de nitrocellulose (dot blot par aspiration). On laisse incuber la membrane dans un tampon radioactif (³²P (10 mCi/ml, Amersham-Biosciences) 2M ; Tris 50 mM ; pH 8,0)
La membrane est rapidement rincée 2x1 min dans un tampon Tris 50 mM, pH 8,0. En exposant un film photographique avec la membrane (environ 45 min) on peut détecter les zones qui fixent le phosphate radioactif (voir Figure 3 ci-après).

La présente invention concerne également une séquence nucléotidique codant pour une protéine telle que définie ci-dessus.

La présente invention concerne également un vecteur recombinant, notamment plasmide, cosmide, phage ou ADN de virus, contenant une séquence nucléotidique telle que définie ci-dessus.

Selon un mode de réalisation avantageux, la présente invention concerne un vecteur recombinant tel que défini ci-dessus, contenant les éléments nécessaires à l'expression dans une cellule hôte des polypeptides codés par la séquence nucléotidique telle que définie ci-dessus, insérée dans ledit vecteur.

La présente invention concerne également une cellule hôte, choisie notamment parmi les bactéries, les virus, les levures, les champignons, les plantes ou les cellules de mammifères, ladite cellule hôte étant transformée, notamment à l'aide d'un vecteur recombinant tel que défini ci-dessus.

La présente invention concerne également une composition pharmaceutique comprenant à titre de substance active une protéine telle que définie ci-dessus, notamment SEQ ID NO : 2 ou SEQ ID NO : 3, en association avec un véhicule pharmaceutiquement acceptable.

La présente invention concerne également une composition pharmaceutique telle que définie ci-dessus, dans laquelle la protéine de l'invention, notamment SEQ ID NO : 2 ou SEQ ID NO : 3, est en association avec un variant de la protéine paraoxonase, ayant une activité d'hydrolyse du paraoxon.

Parmi les variants de la paraoxonase, on peut citer les variants PON1, PON2, PON3, d'origine humaine ou non, tels que SEQ ID NO : 4 (PON1 humaine ; Hassett et al., 1991), SEQ ID NO : 5 (PON2 humaine ; Primo-Parmo et al., 1996), SEQ ID NO : 6 (PON3 humaine ; Reddy et al., 2001), SEQ ID NO : 7 (PON1 de lapin ; Hassett et al., 1991), SEQ ID NO : 8 (PON1 de rat ; Rodrigo et al., 1997), SEQ ID NO : 9 (PON1 de souris ; Sorenson et al., 1995), SEQ ID NO : 10 (PON2 de souris ; Primo-Parmo et al., 1996) et SEQ ID NO : 11 (PON3 de souris ; Primo-Parmo et al., 1996).

La présente invention concerne également l'utilisation d'une protéine telle que définie ci-dessus, notamment SEQ ID NO : 2 ou SEQ ID NO : 3, pour la préparation d'un médicament destiné à la prévention ou au traitement de maladies liées à une hyperphosphatémie, telles que les maladies cardiovasculaires et l'arthrite (pseudo-goutte).

Le terme "hyperphosphatémie" désigne un excès de phosphate dans l'organisme.
Plus exactement, l'hyperphosphatémie est définie par une augmentation de la concentration plasmatique de phosphate au dessus de 1,44 mmol/l (45 mg/l), ladite quantité étant obtenue par dosage du phosphate total (le dosage par méthode colorimétrique est effectué après un procédé de minéralisation).

Selon un mode de réalisation avantageux, la protéine de l'invention pourra être administrée sous forme intraveineuse pour pouvoir fixer une quantité maximale de phosphate pendant une longue période, de l'ordre de la semaine. En éliminant ultérieurement la protéine, une grande quantité de phosphate sera ainsi éliminée rapidement. Ceci permet d'espacer et diminuer les temps de dialyse.

La présente invention concerne plus particulièrement l'utilisation d'une protéine telle que définie ci-dessus, notamment SEQ ID NO : 2 ou SEQ ID NO : 3, dans le cadre de la prévention ou du traitement des maladies cardiovasculaires.

La présente invention concerne également l'utilisation d'une protéine selon l'invention, notamment de la protéine représentée par la séquence SEQ ID NO : 2 ou SEQ ID NO : 3, en association avec une protéine telle qu'un variant de la protéine paraoxonase, dans le cadre de la prophylaxie ou du traitement des intoxications provoquées par des insecticides ou des agents neurotoxiques, tels que le soman, le VX, le tabun ou le sarin, ou dans le cadre du traitement de l'athérosclérose.

La présente invention concerne également un produit de combinaison comprenant au moins une protéine telle que définie ci-dessus, notamment SEQ ID NO : 2 ou SEQ ID NO : 3, et au moins un variant de la protéine paraoxonase, pour une utilisation simultanée, séparée ou étalée dans le temps destiné à la prophylaxie ou au traitement des intoxications provoquées par des insecticides ou des agents neurotoxiques, tels que le soman, le VX, le tabun ou le sarin.

L'utilisation combinée de la protéine de l'invention, notamment SEQ ID NO : 2, avec un variant de la protéine paraoxonase, permet d'accroître la stabilité de la paraoxonase, notamment dans le cadre de la prophylaxie ou du traitement des intoxications provoquées par des insecticides ou des agents neurotoxiques.

La présente invention concerne également une méthode de dosage de la protéine telle que définie ci-dessus, caractérisée en ce qu'elle comprend les étapes suivantes :
- des anticorps monoclonaux de lapin dirigé contre différents épitopes de la protéine de l'invention (anti-HPB) sont fixés sur une plaque et le sérum humain à analyser contenant ladite protéine (HPB) est déposé sur la plaque susmentionnée,
- la plaque est rincée et lavée,
- on dépose sur la plaque des anticorps anti-anticorps de lapin (anti-IGrabbit-per) marqués avec de la peroxydase durant 30 minutes, afin de former un complexe ternaire entre un anticorps monoclonal de lapin, la protéine selon l'invention et un anticorps anti-anticorps de lapin susmentionnés (anti-HPB - HPB - anti-IGrabbit-per),
- la plaque est rincée et lavée,
- on fait réagir la peroxydase fixée sur la plaque avec son substrat (kit disponible en commerce, Chemiluminescent Peroxidase Substrate (Sigma)) et la réaction est arrêtée au bout de 30 minutes avec la 3,3',5,5'-tétraméthylbenzidine (TMB, Sigma),
- la densité optique du produit formé à l'étape précédente est mesurée à 450 nm à l'aide d'un spectrophotomètre, et la comparaison de cette mesure avec une courbe étalon permet de déterminer la concentration de la protéine selon l'invention (HPB) présente dans le sérum.

Ainsi, la méthode de dosage susmentionnée utilise une méthode par immunodosage du type ELISA (Engvall et al., 1971).

D'autres méthodes peuvent être utilisées pour doser la concentration de la protéine de l'invention dans le plasma telles que :
- les méthodes électrophorétiques, ou
- la quantification de son activité.

La présente invention concerne également l'application de la méthode de dosage telle que définie ci-dessus
au diagnostic *in vitro* de maladies liées à une hyperphosphatémie notamment lorsque la quantité de protéine telle que définie ci-dessus, notamment SEQ ID NO : 2 ou SEQ ID NO : 3, dosée selon la méthode telle que définie ci-dessus, est inférieure à la quantité de cette protéine normalement présente dans le sang d'un individu sain, ou
au diagnostic *in vitro* de maladies liées à une hypophosphatémie notamment lorsque la quantité de protéine telle que définie ci-dessus, notamment SEQ ID NO : 2 ou SEQ ID NO : 3, dosée selon la méthode telle que définie ci-dessus, est supérieure à la quantité de cette protéine normalement présente dans le sang d'un individu sain, ou
au diagnostic *in vitro* d'une prédisposition d'un individu à de telles pathologies.

Le taux de la protéine selon l'invention est un indicateur de prédisposition à un risque de maladie cardiovasculaire. Ainsi, les personnes ayant un taux faible de ladite protéine auront un taux plus important de phosphate libre qui précipitera avec le calcium du plasma pour former des plaques de phosphate de calcium, ce qui est un facteur aggravant notamment les risques de maladies cardiovasculaires ou d'arthrite.

Un taux anormal de cette protéine est aussi le signe d'une pathologie existante. Par exemple une hyperphosphatémie peut déclencher une production accrue de protéine dans le but de limiter le taux de phosphate. Un taux faible peut être lui aussi révélateur d'un dysfonctionnement.

La présente invention concerne également l'application telle que définie ci-dessus au diagnostic *in vitro* de maladies liées à une hyperphosphatémie telles que les maladies cardiovasculaires, notamment les maladies cardiovasculaires liées à la formation de plaques d'athéromes, ou au diagnostic *in vitro* d'une prédisposition d'un individu au développement d'une des maladies susmentionnées.

La présente invention concerne également l'application telle que définie ci-dessus au diagnostic *in vitro* de maladies liées à une hypophosphatémie, ou au diagnostic in *vitro* d'une prédisposition d'un individu au développement de ces maladies.

Parmi les signes cliniques ou physiologiques caractérisant les maladies liées à une hypophosphatémie, on peut citer :
- une déminéralisation des os,
- les manifestations musculaires de l'hypophosphatémie qui comportent une myopathie proximale affectant le muscle squelettique et une dysphagie et un iléus affectant les muscles lisses,
- des carences cardiopulmonaires par le manque d'ATP, et
- une encéphalopathie métabolique.

### LEGENDES DES FIGURES

La Figure 1 représente un gel SDS-PAGE des fractions finales dans le cadre de la purification de la paraoxonase humaine et de la protéine de l'invention SEQ ID NO : 2.
   La colonne A correspond au marqueur de poids moléculaire et les colonnes B, C et D à trois purifications différentes issues de différentes poches de plasma humain. Elles contiennent toutes les trois la paraoxonase humaine et la protéine de fixation du phosphate.
La Figure 2 représente la structure schématique de la protéine de l'invention SEQ ID NO : 2 à laquelle est fixée une molécule de phosphate.
La Figure 3 correspond à un test de fixation du phosphate par la protéine de l'invention SEQ ID NO : 2.
   Les colonnes A à F correspondent à différents lots de purification de la protéine de l'invention provenant de différentes poches de plasma humain ; la colonne G au lysozyme 1 mg/ml et la colonne H à la β-lacto globuline.
La Figure 4 représente un gel bidimensionnel d'électrophorèse du mélange de la protéine de l'invention SEQ ID NO : 2 et de la paraoxonase.

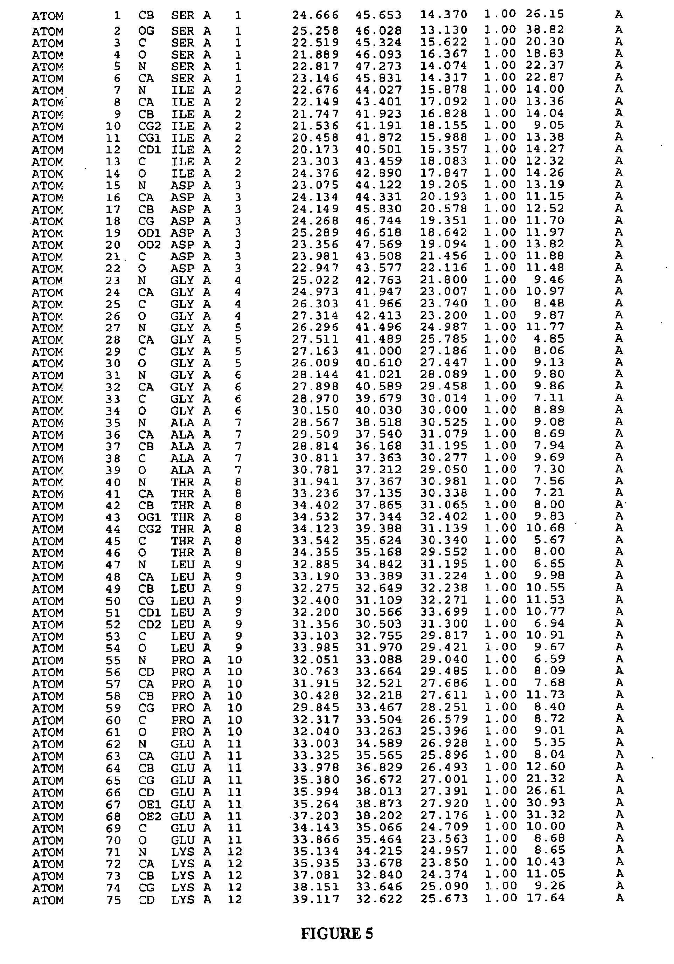

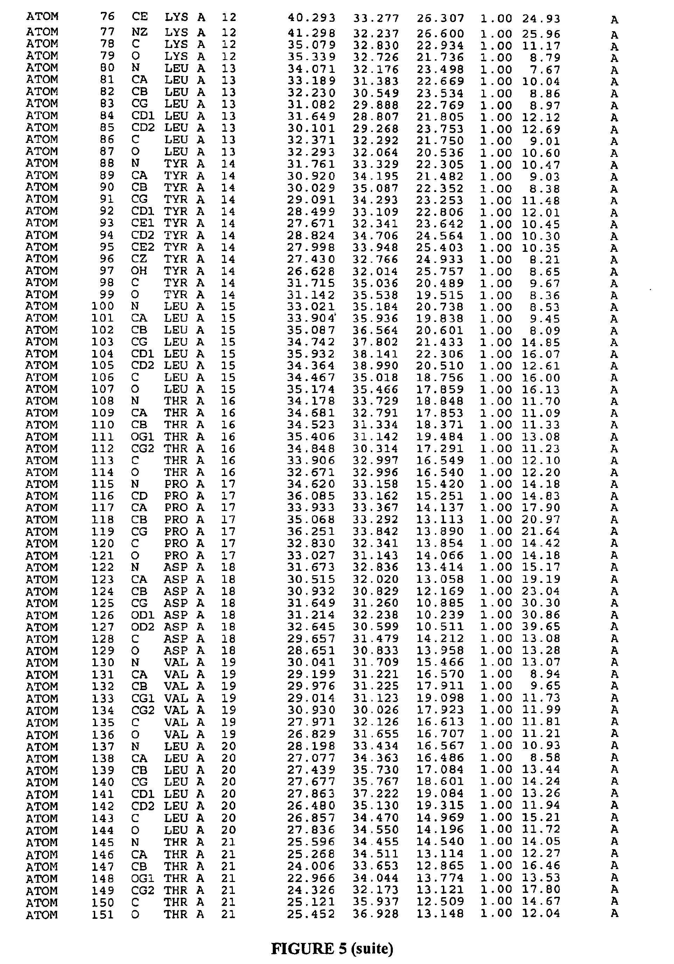

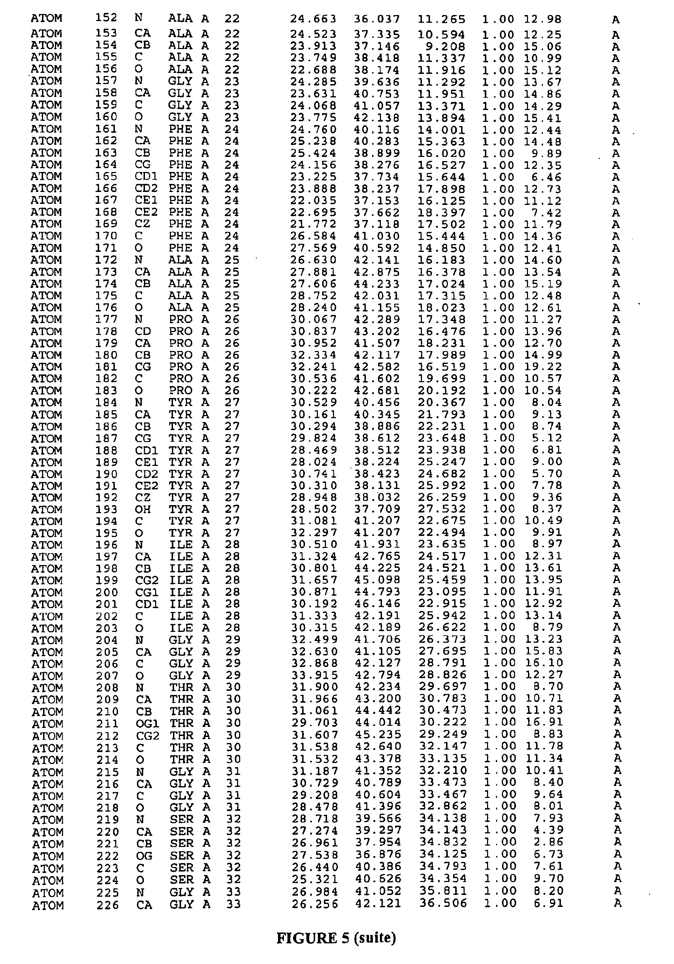

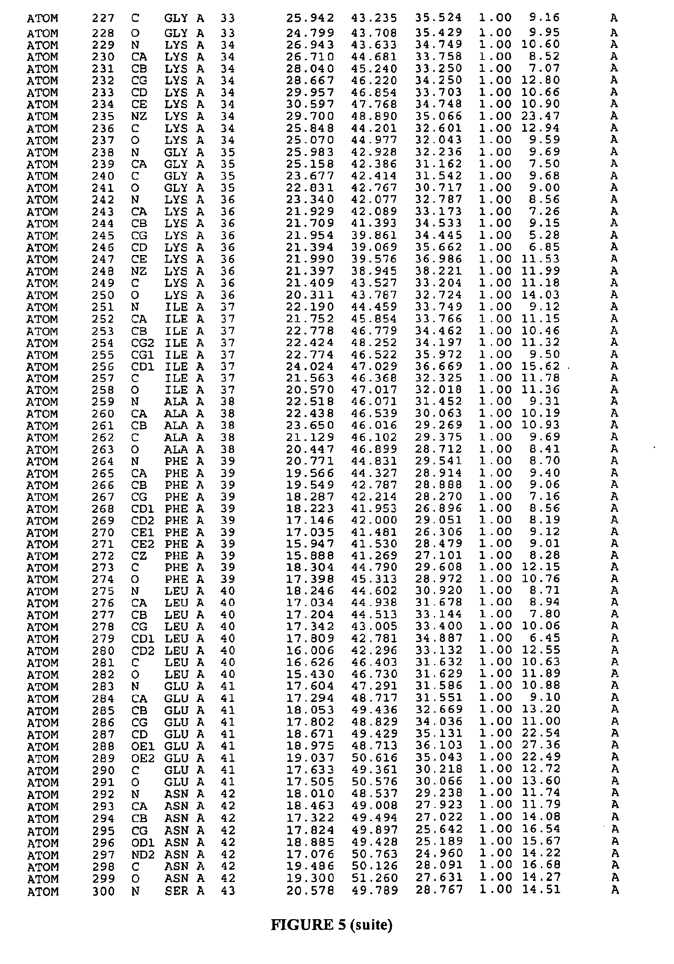

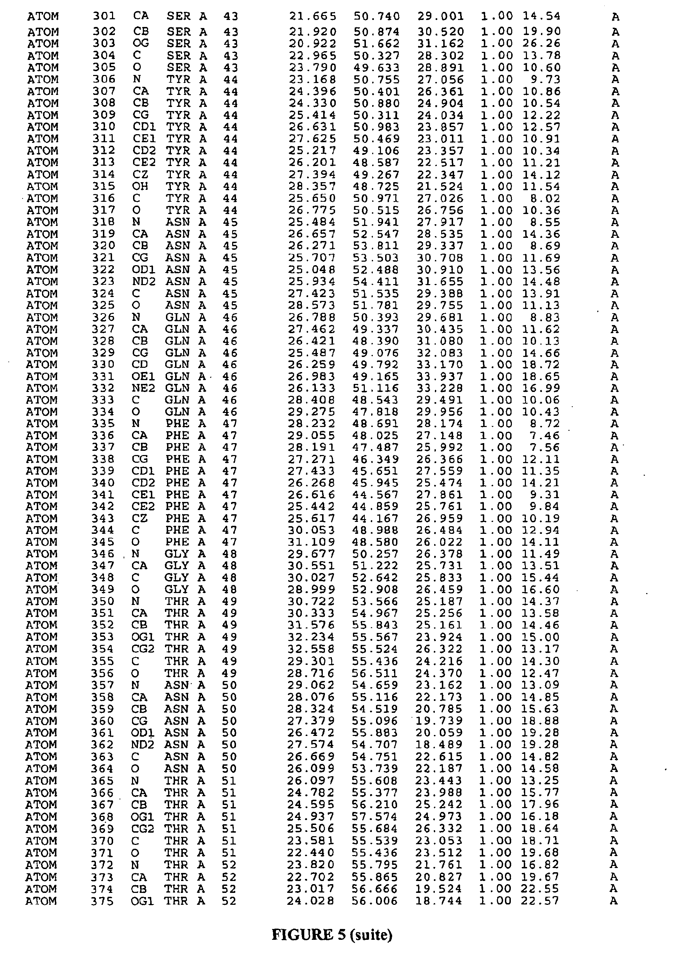

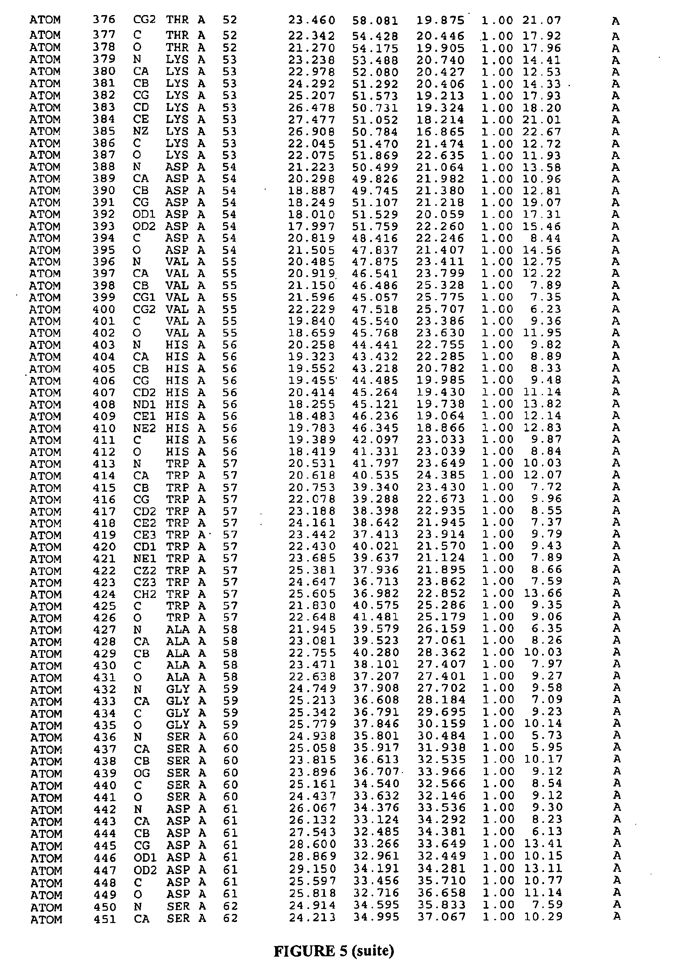

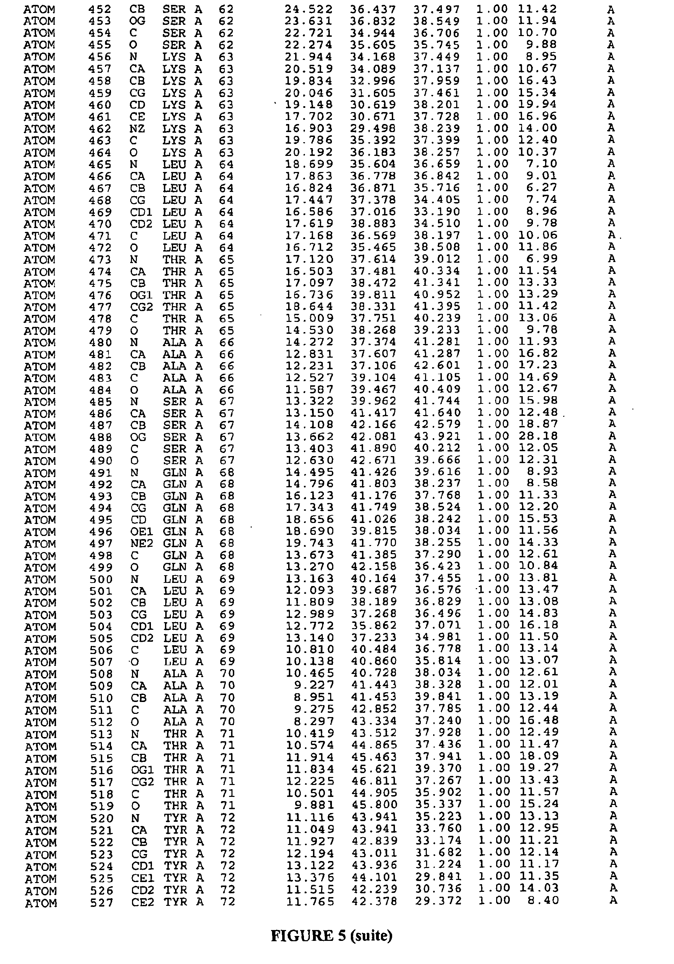

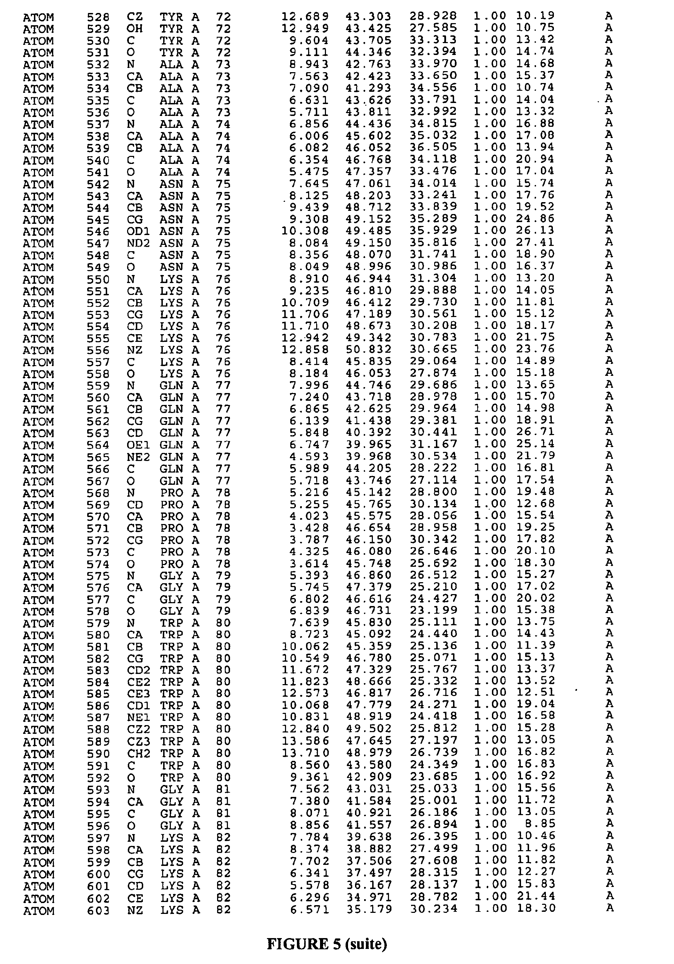

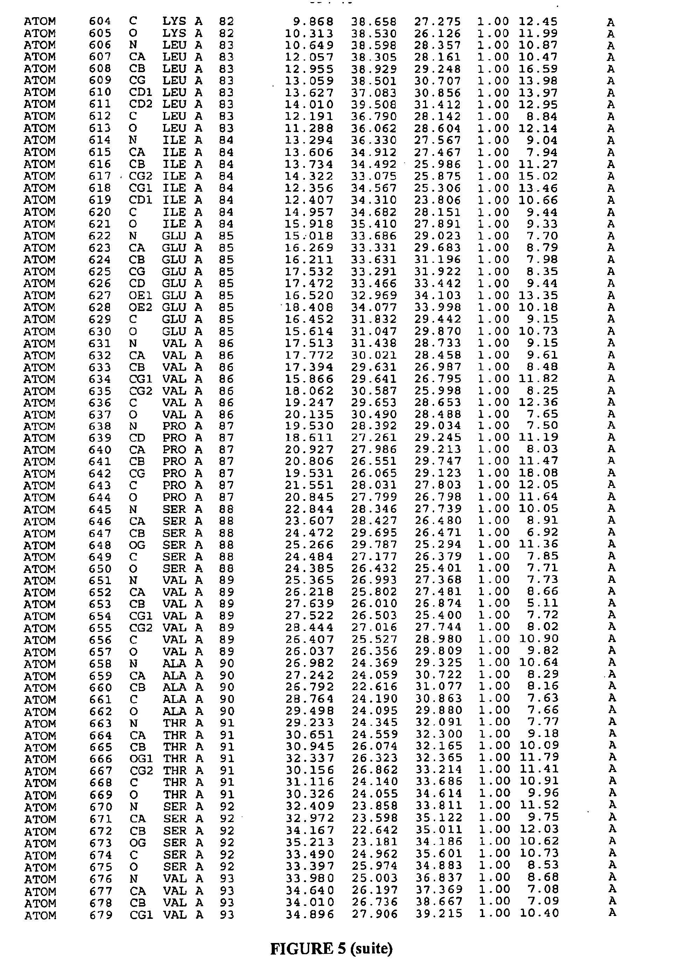

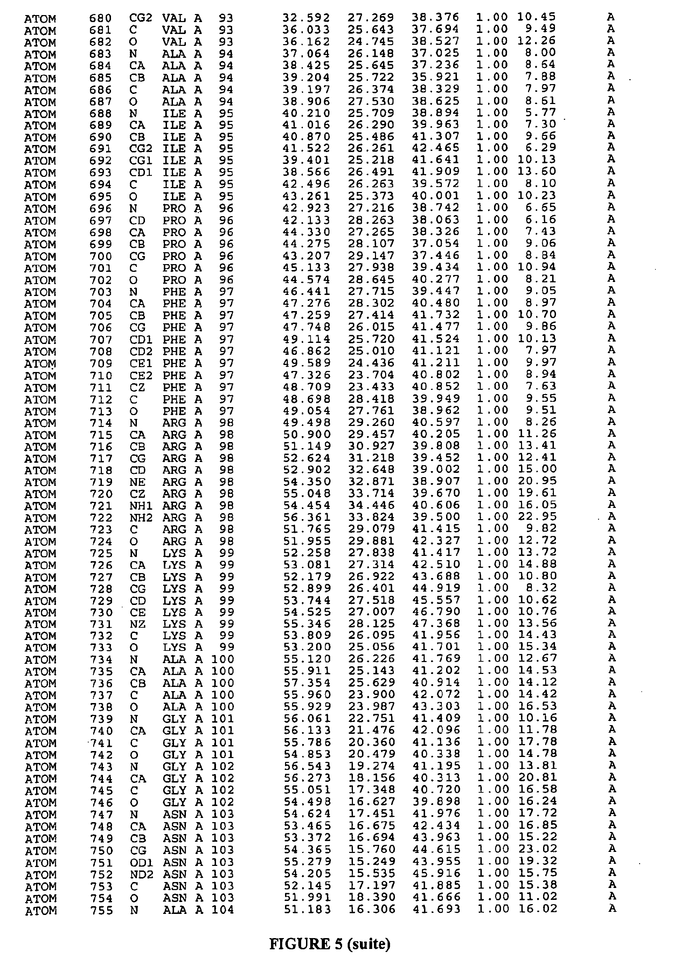

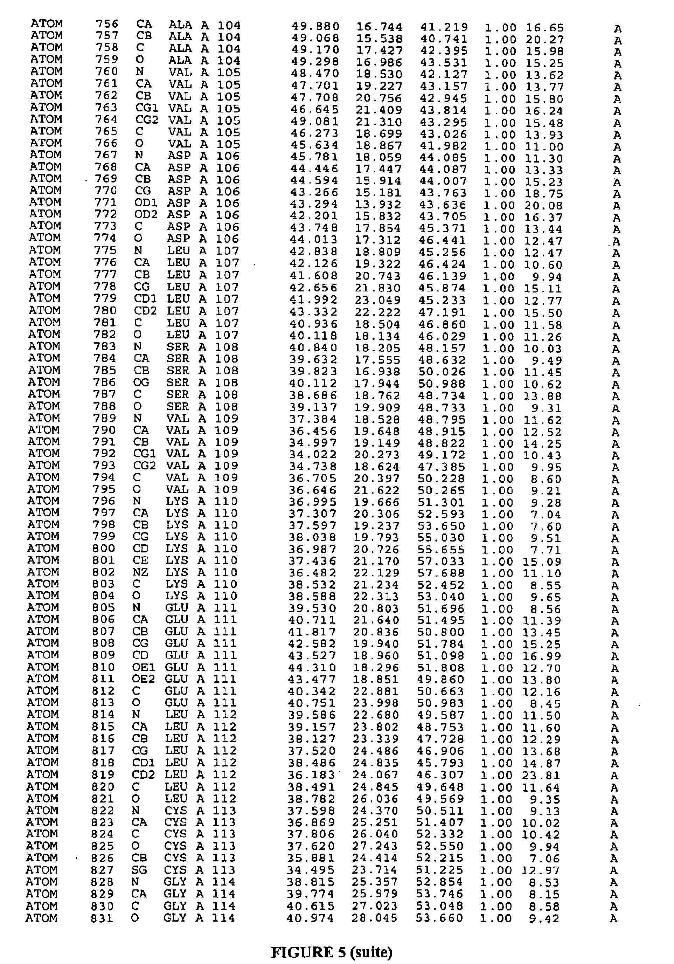

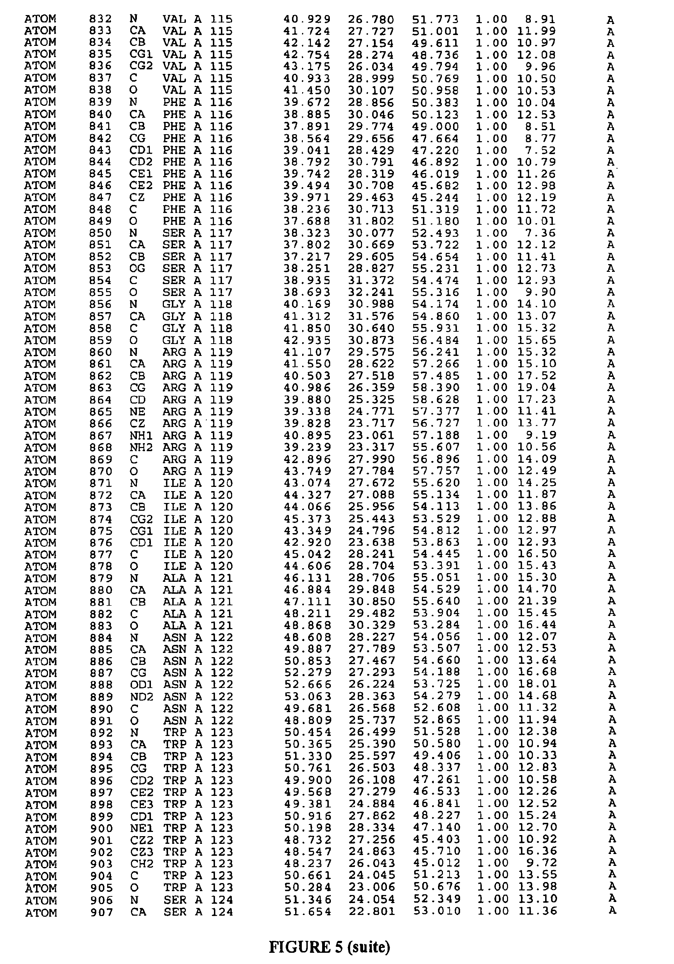

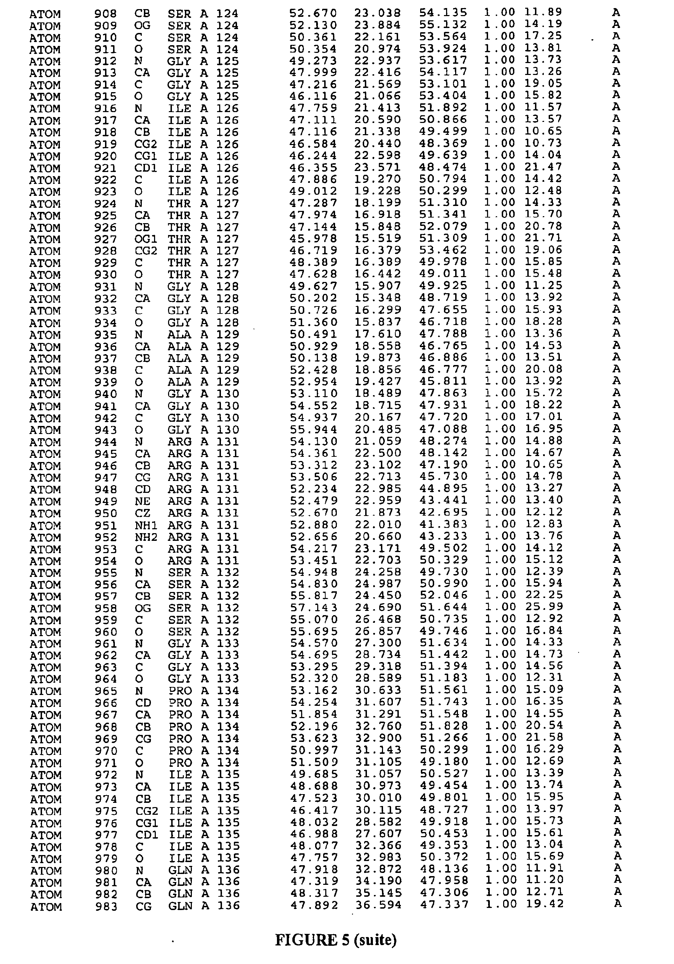

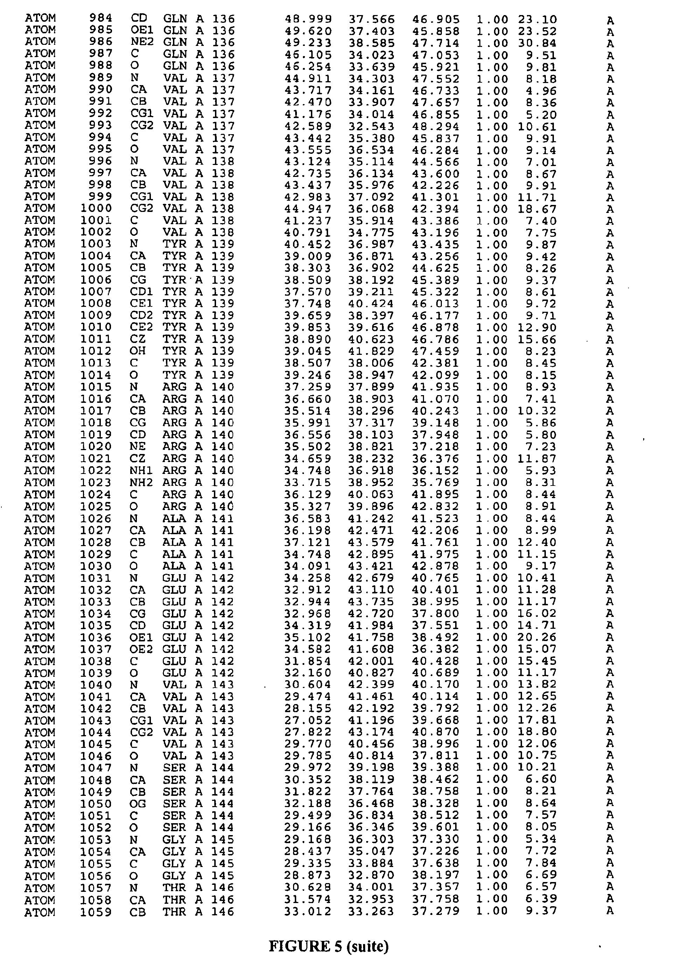

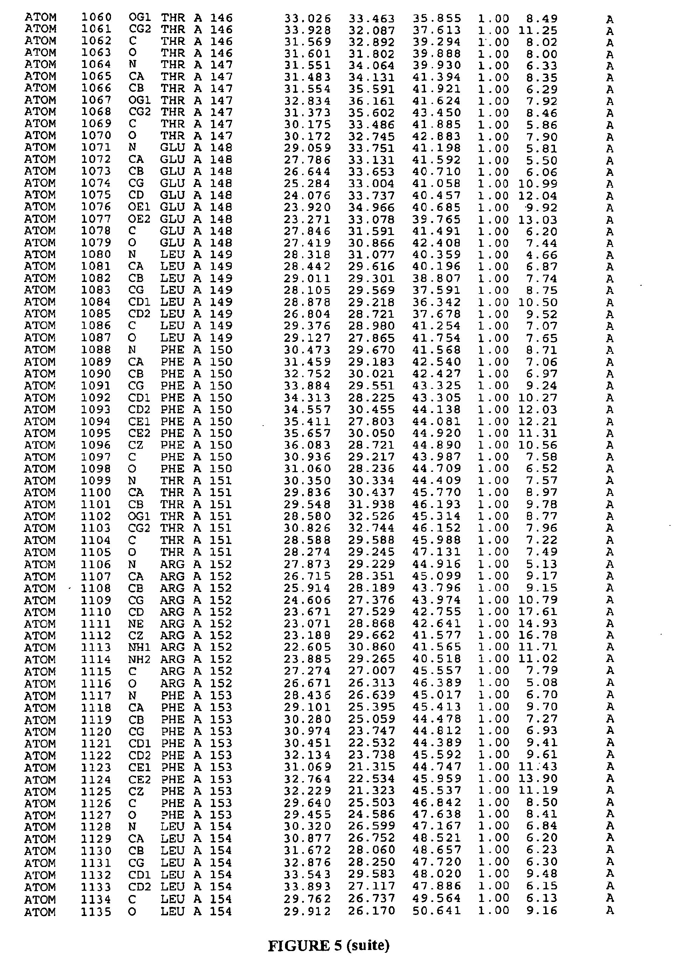

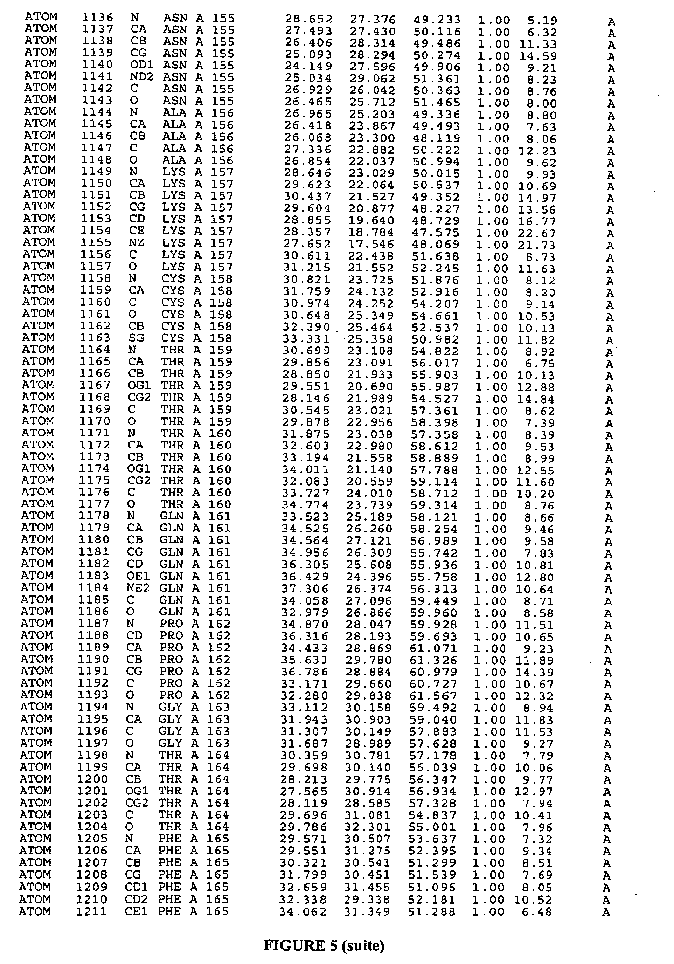

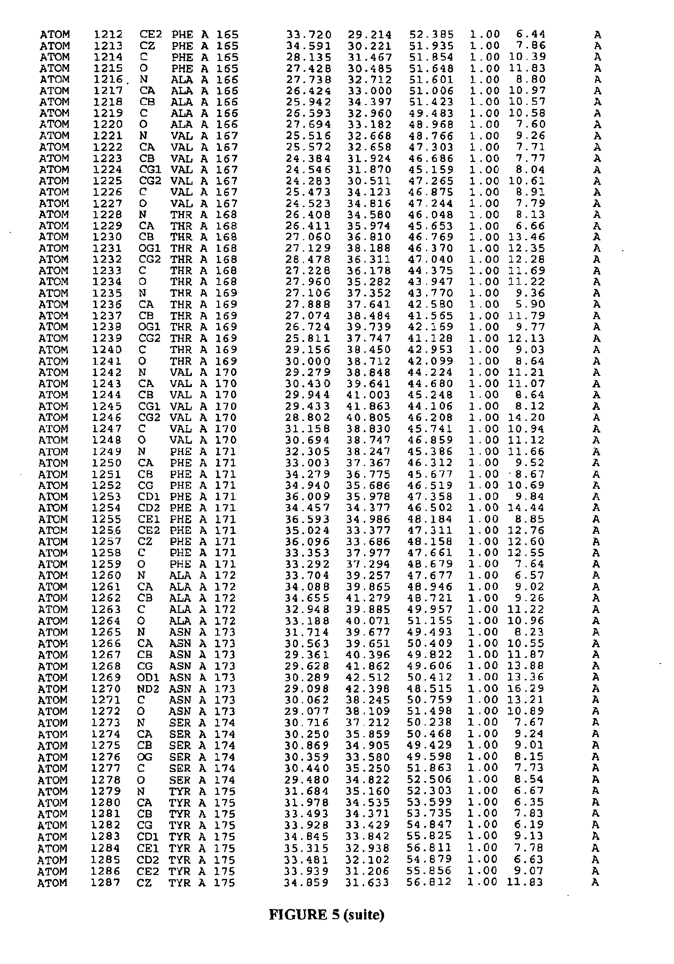

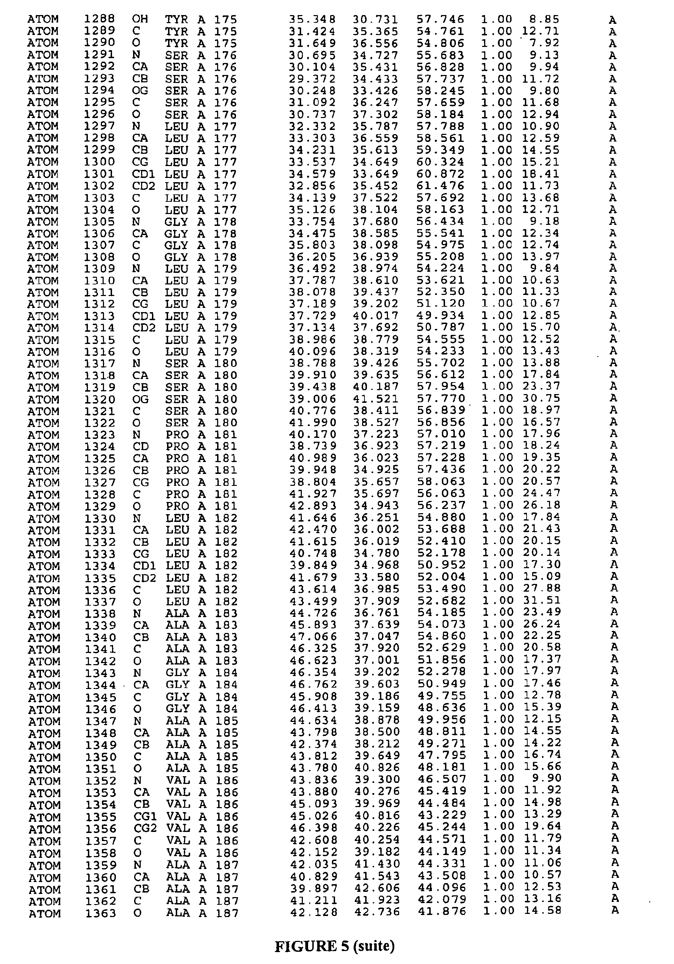

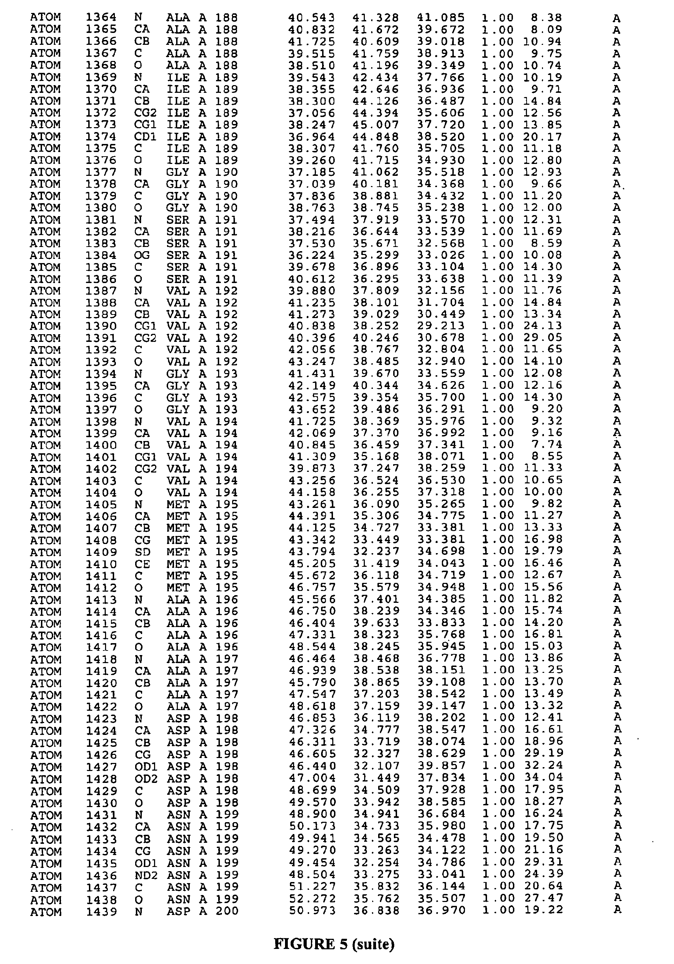

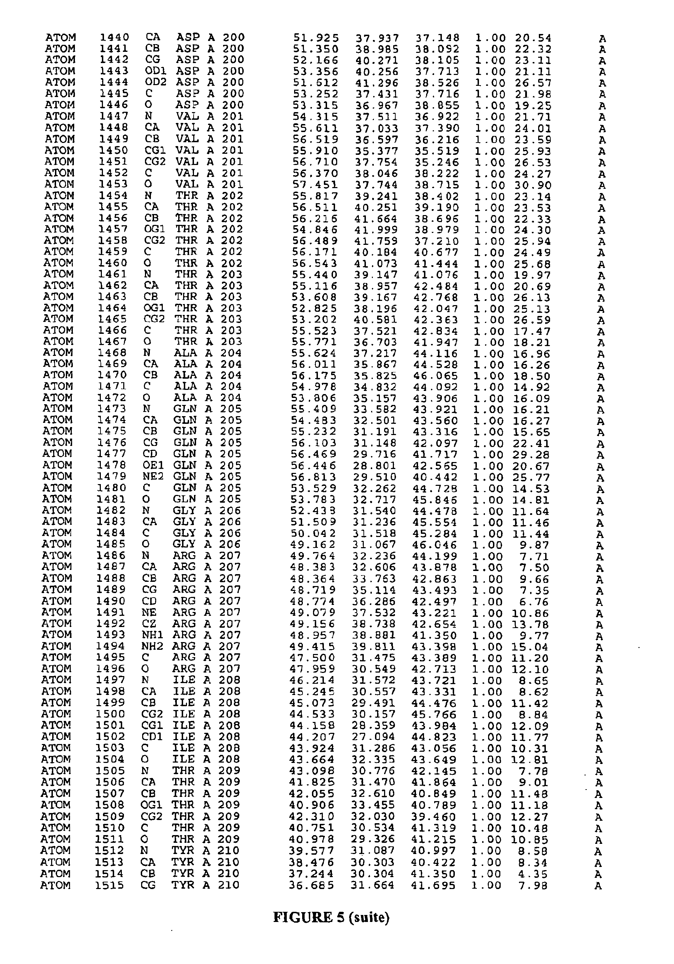

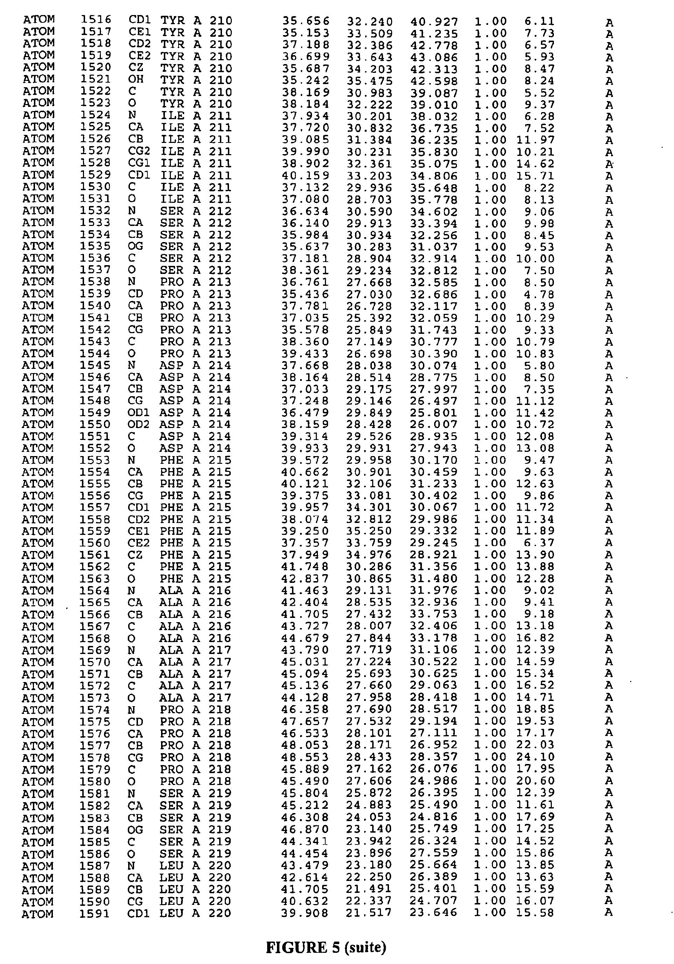

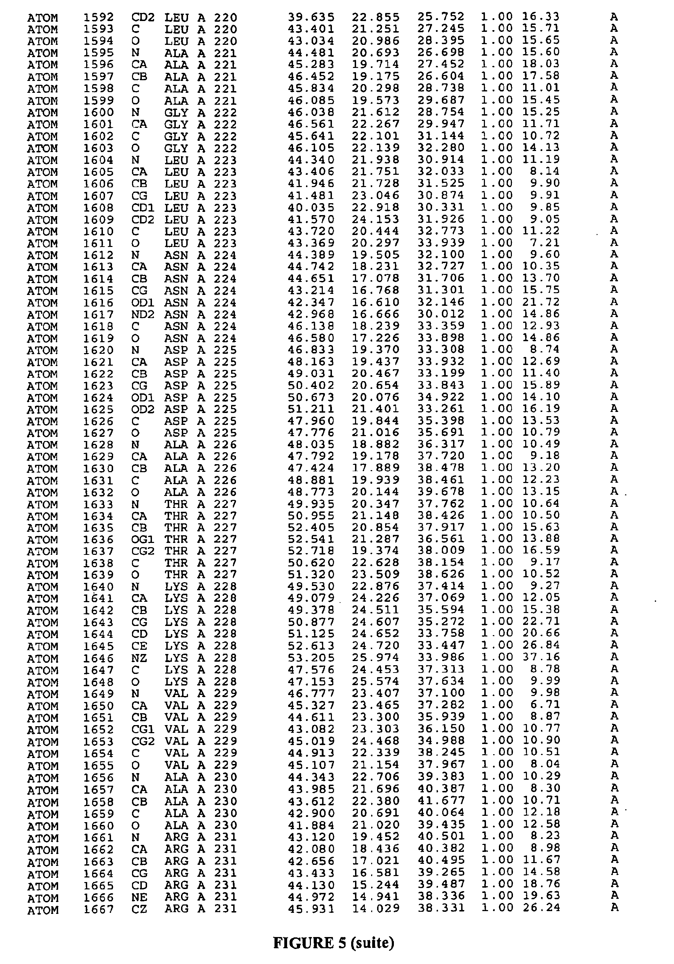

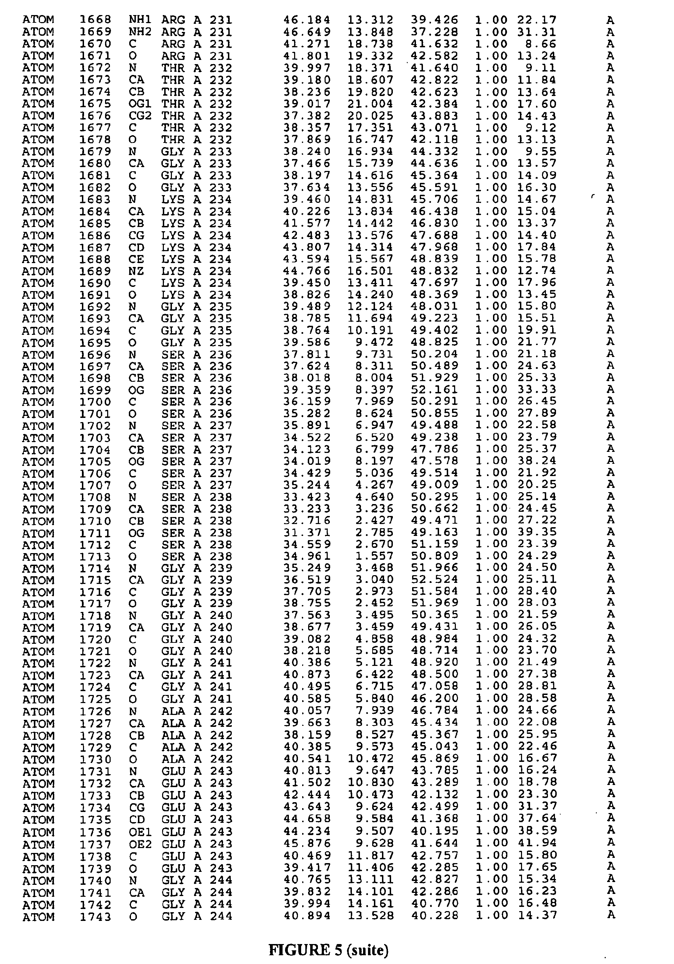

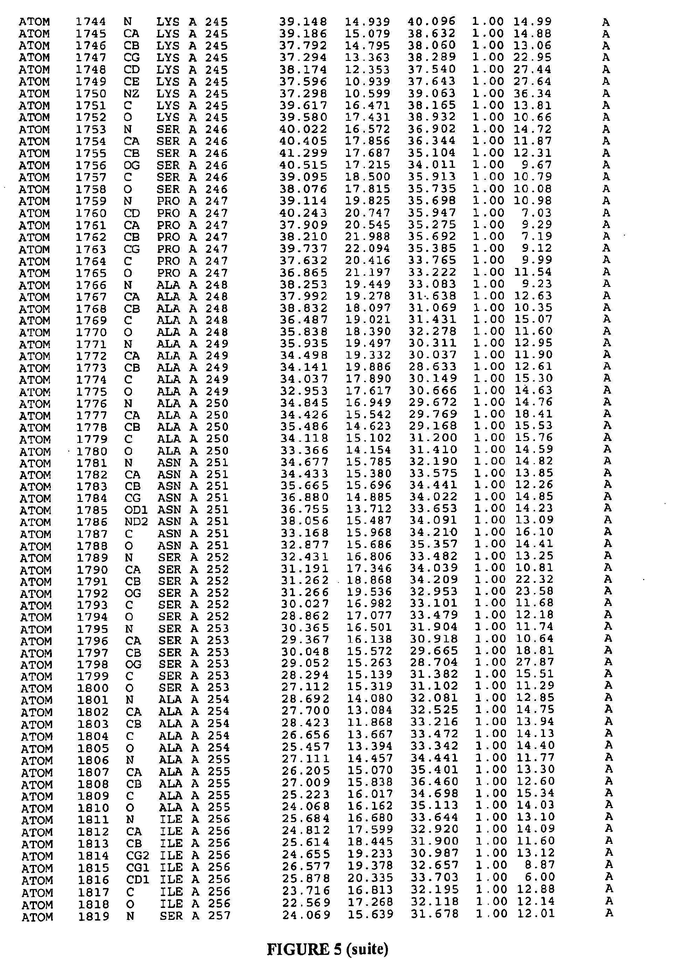

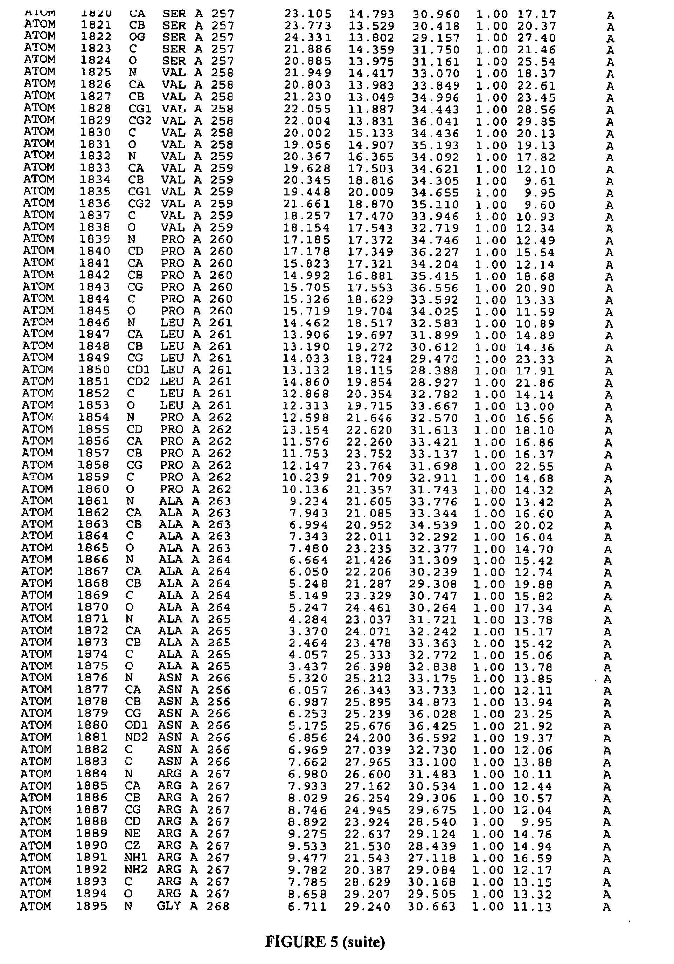

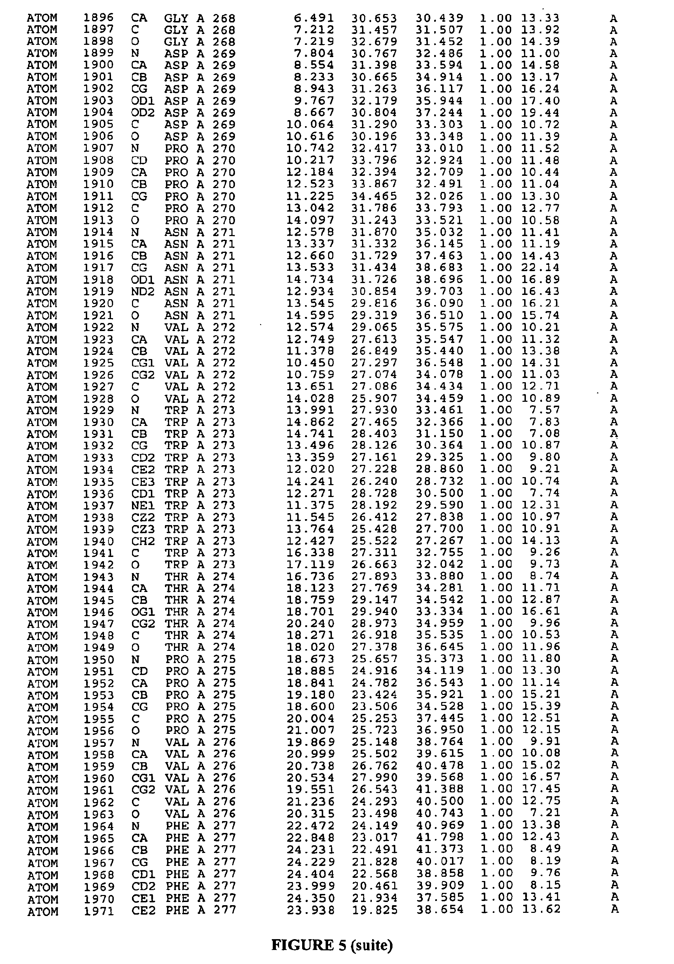

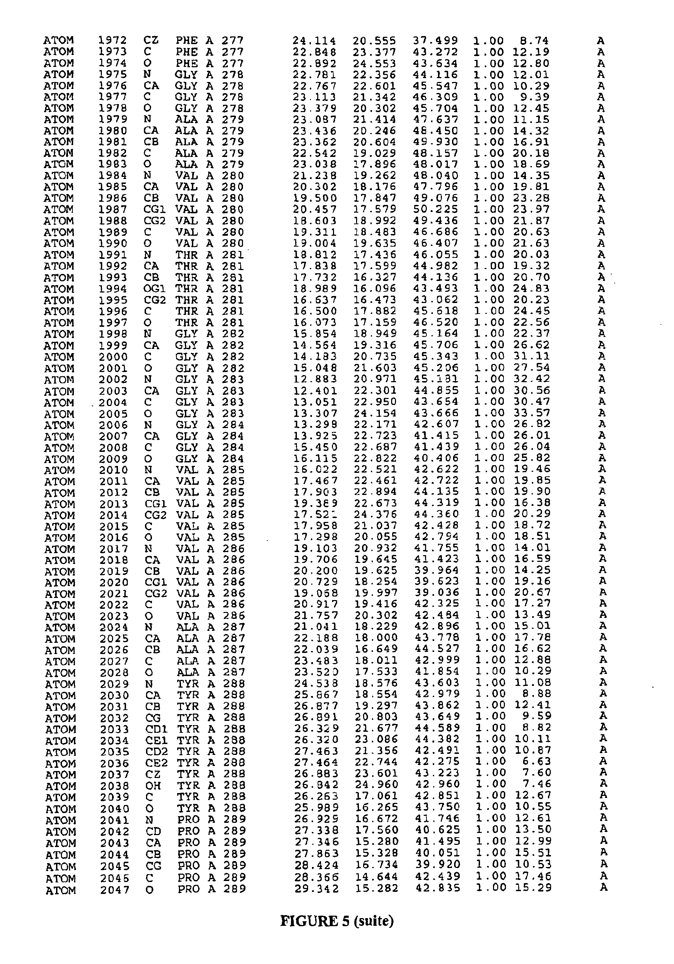

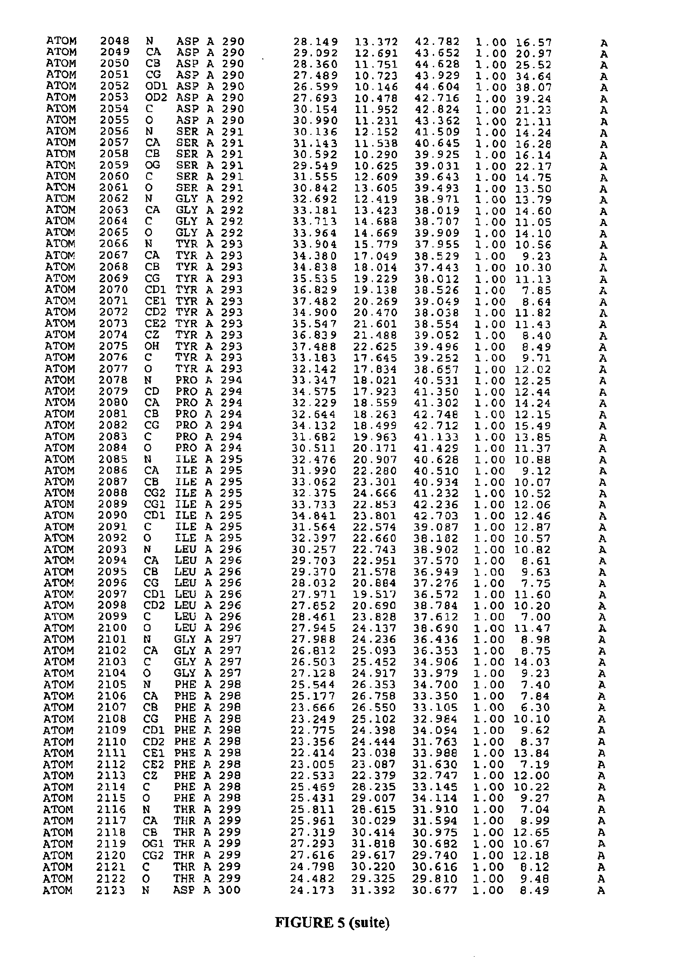

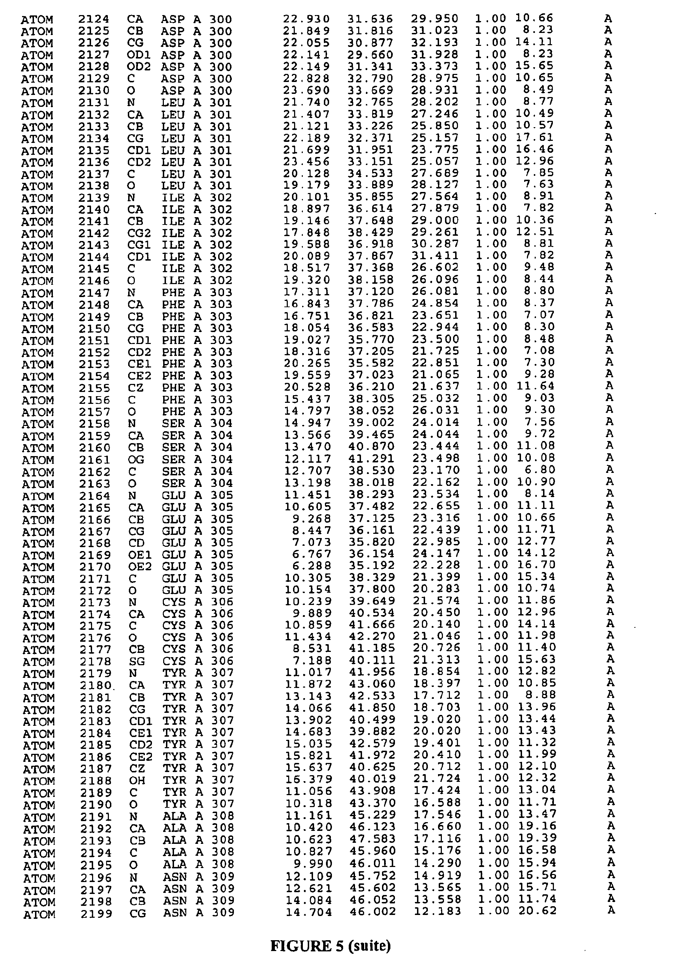

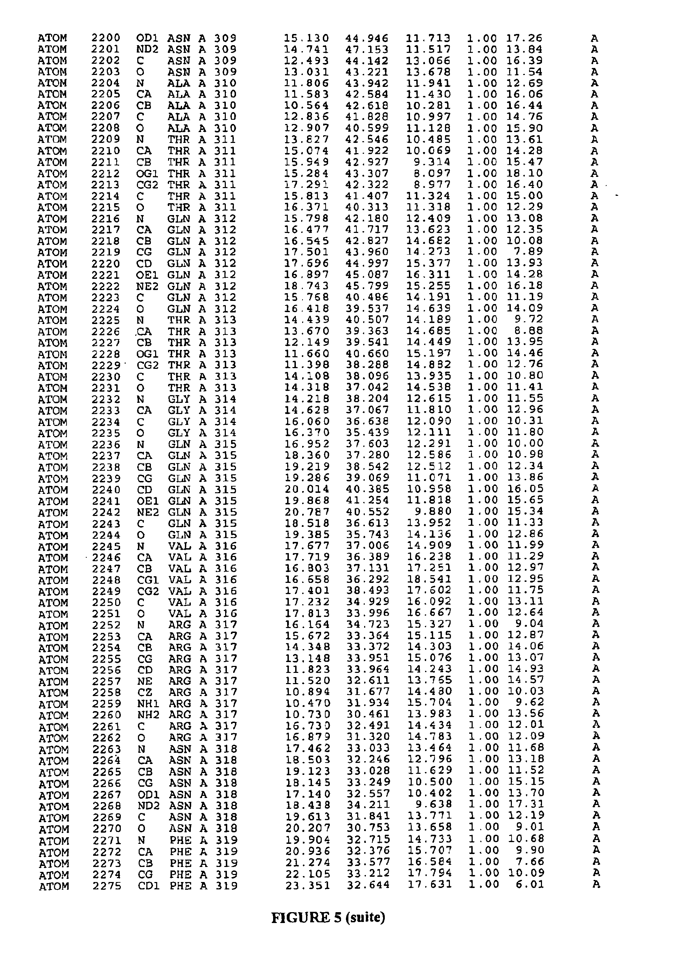

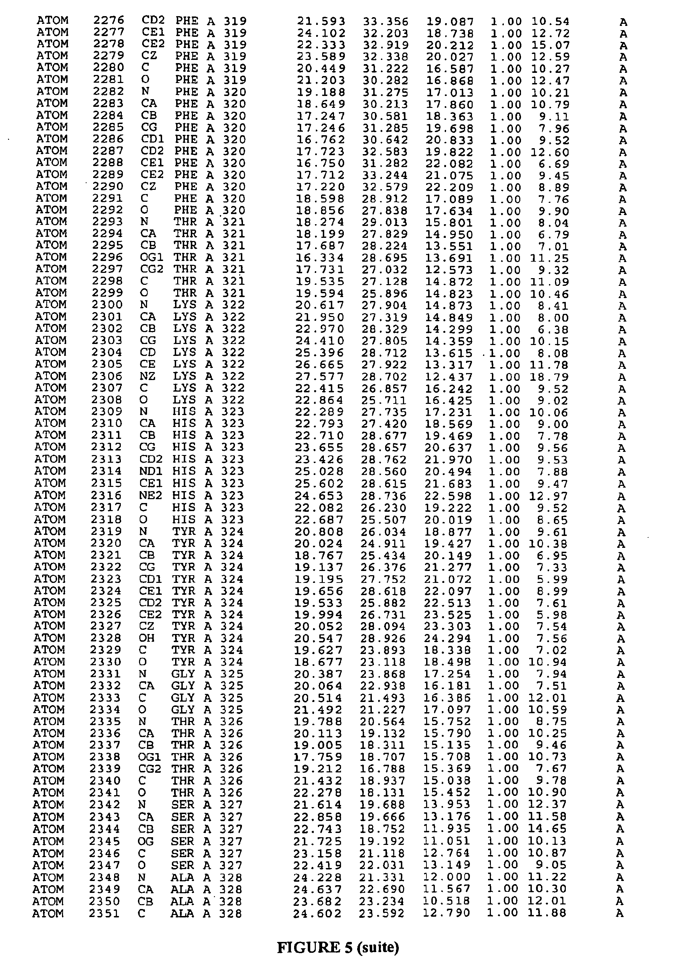

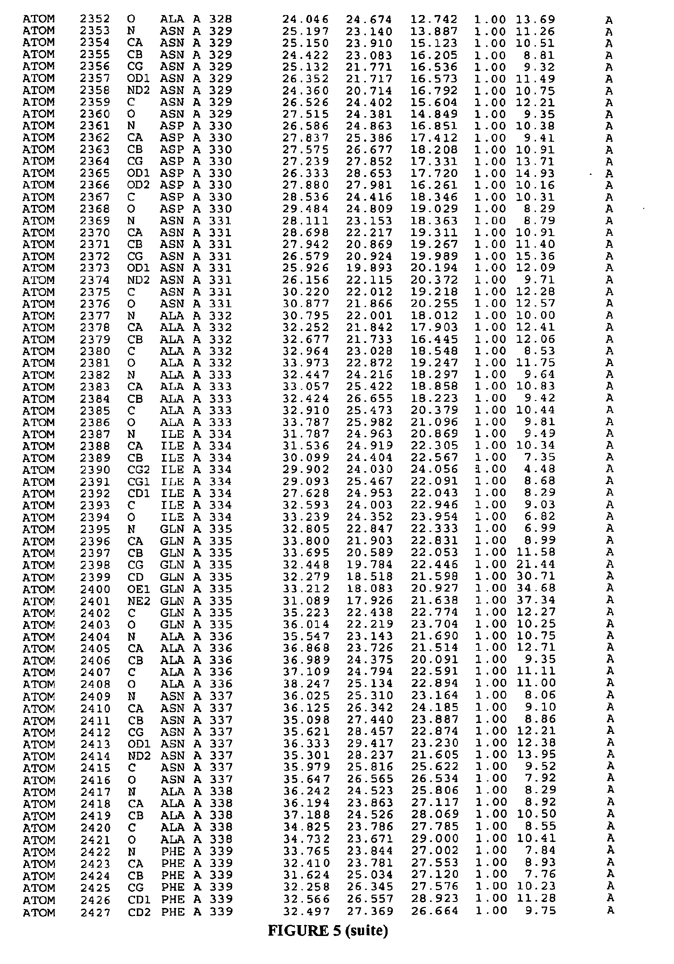

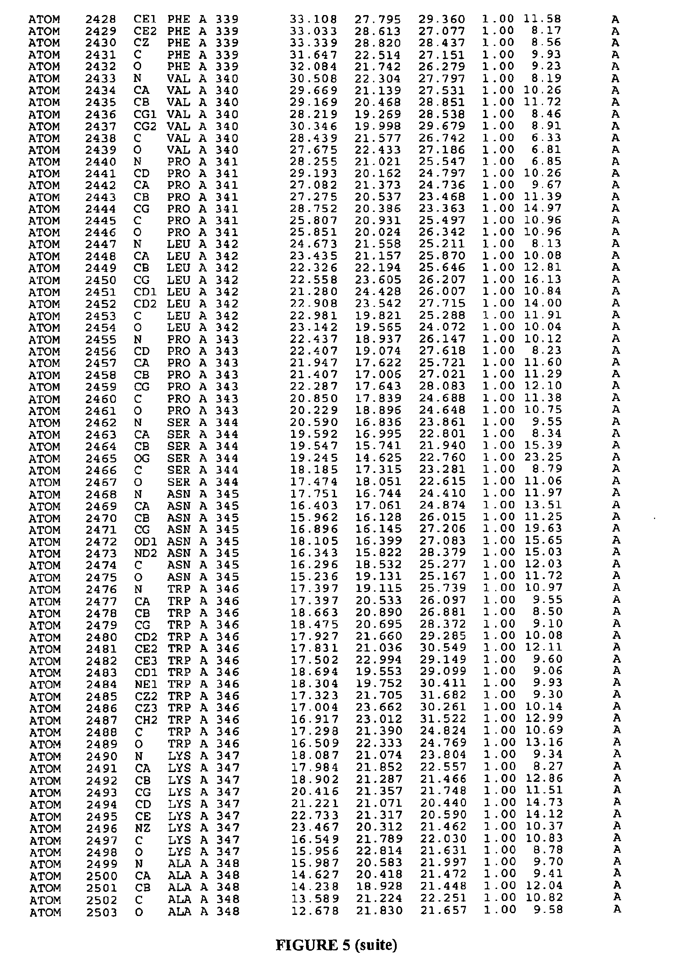

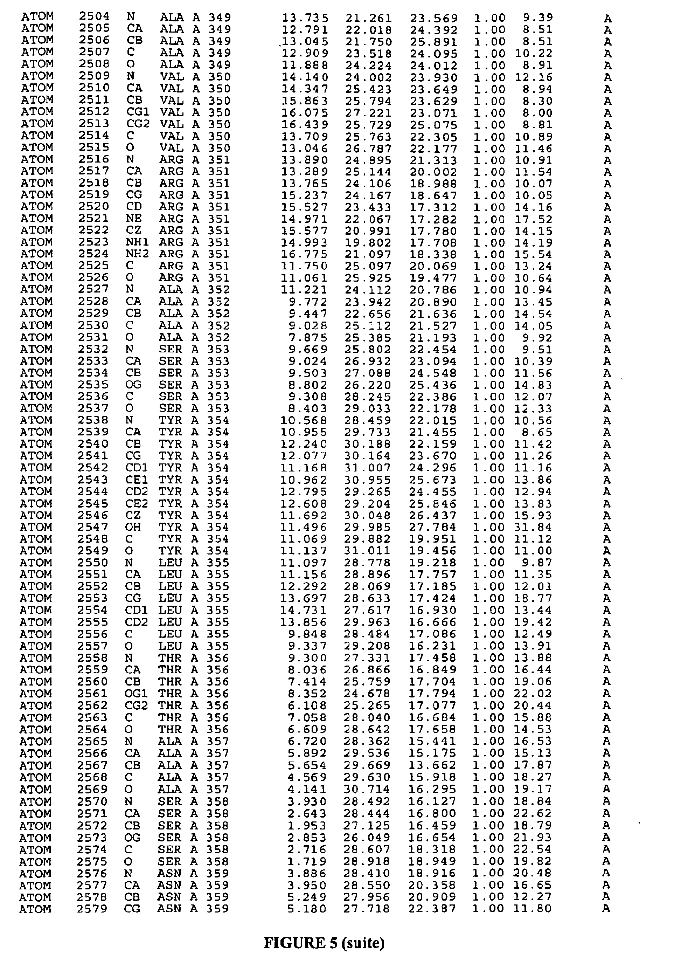

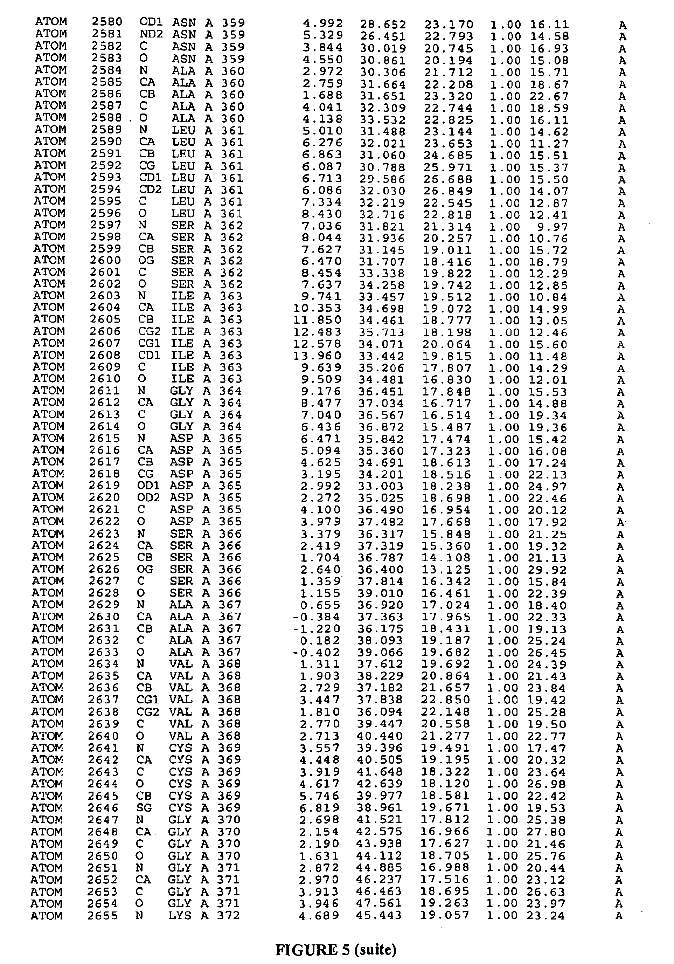

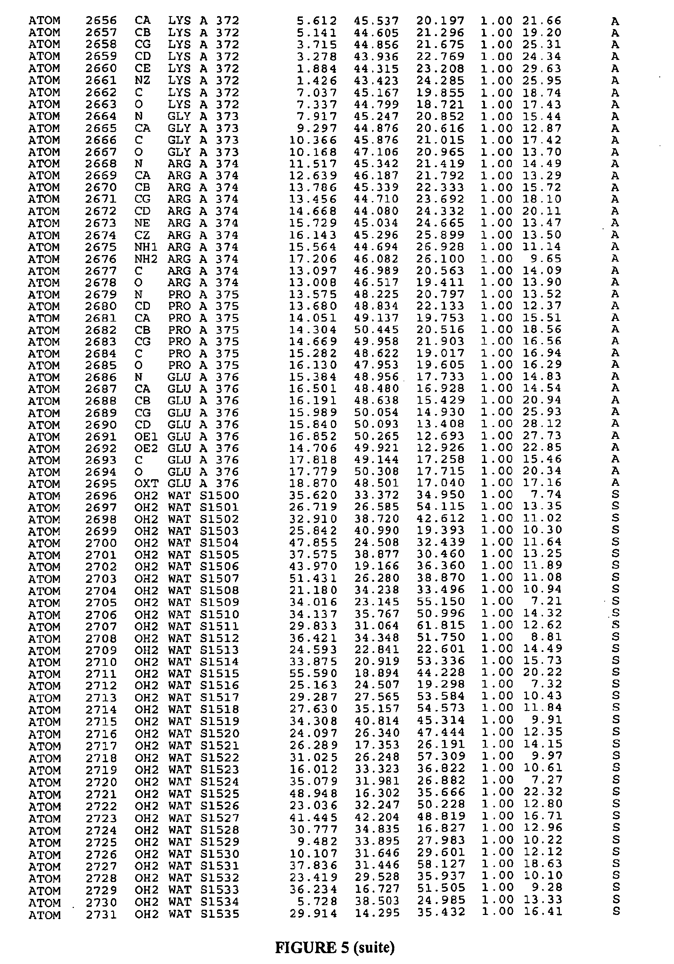

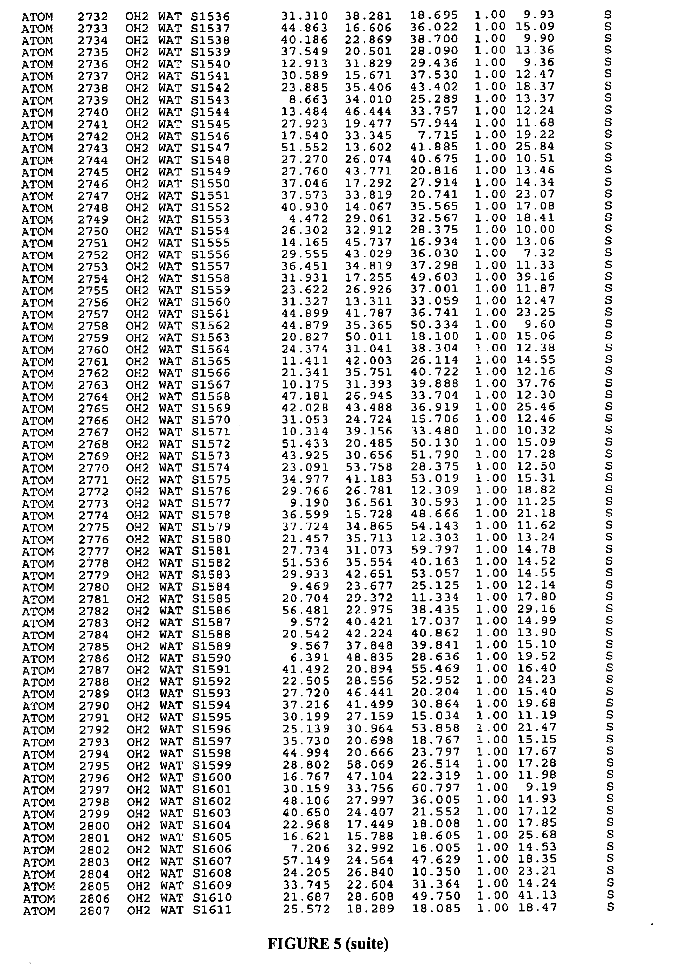

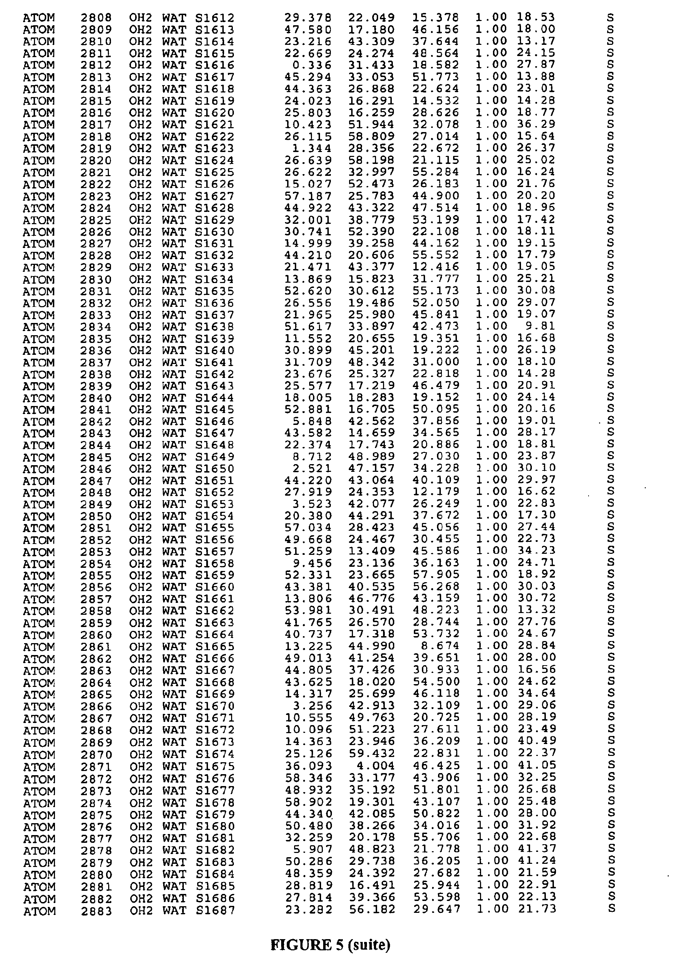

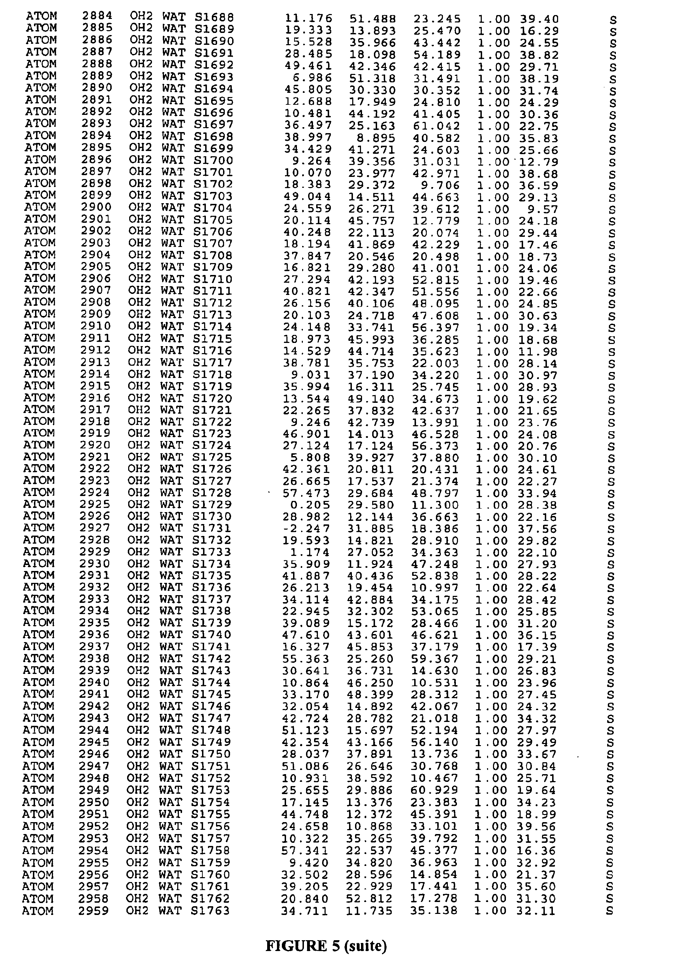

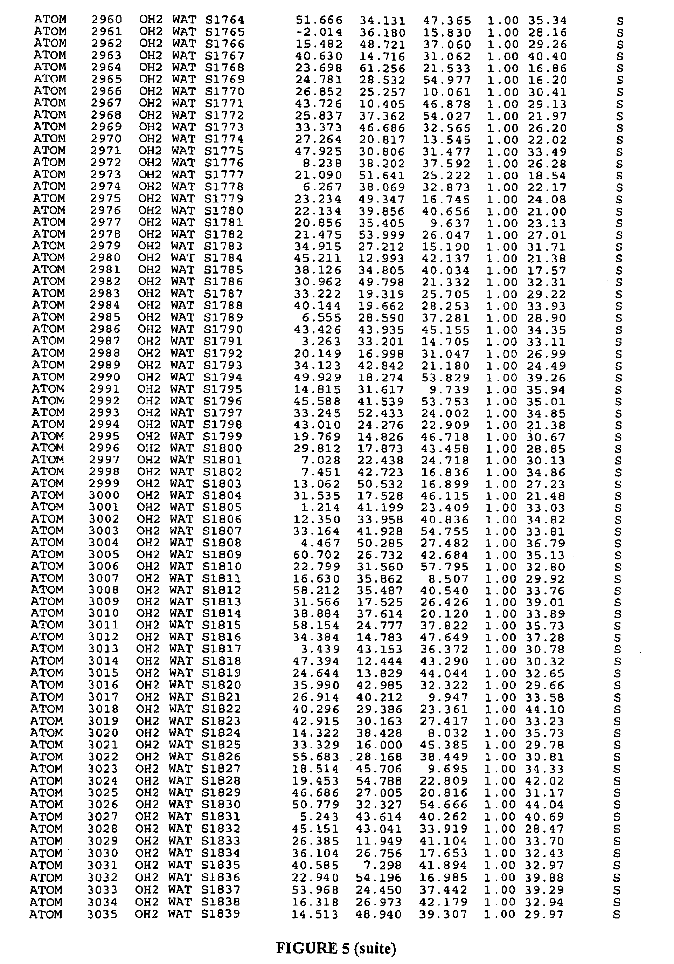

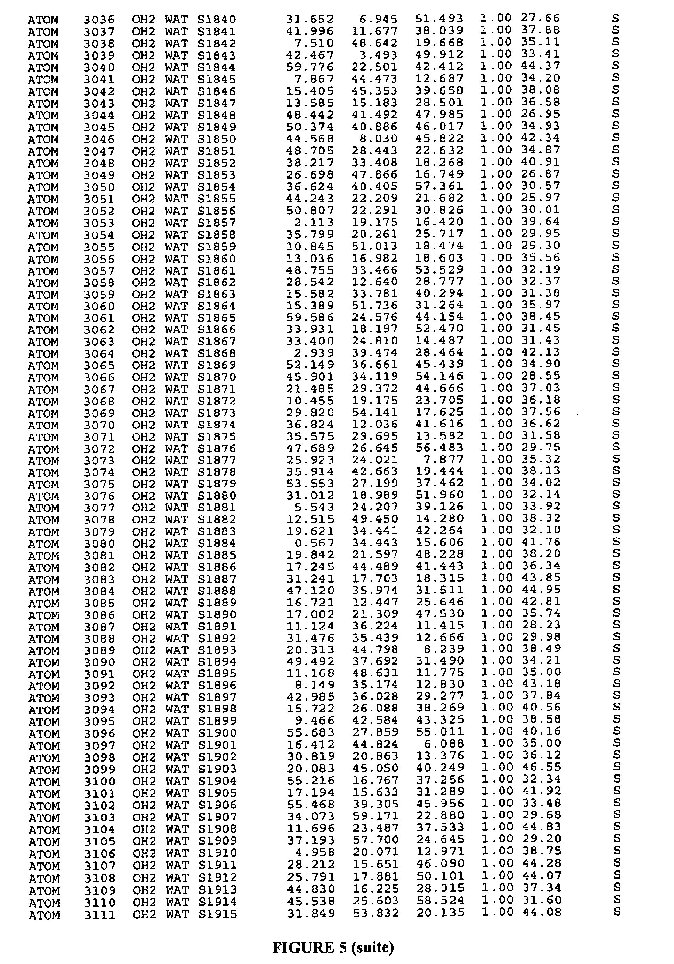

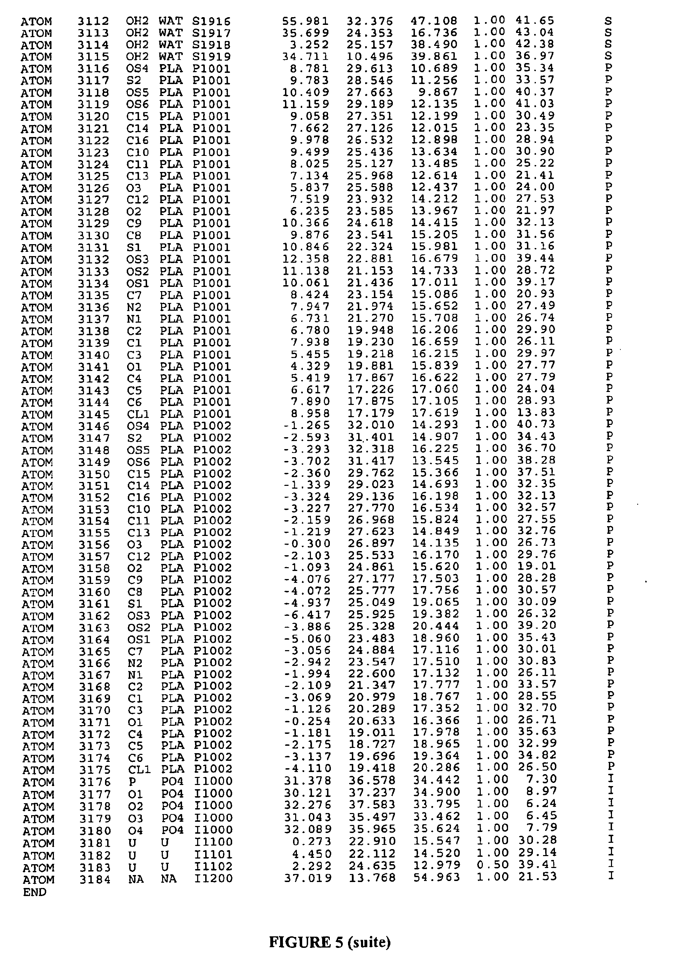

La Figure 5 représente les coordonnées moléculaires de la protéine cristallisée de l'invention SEQ ID NO : 2.

### PARTIE EXPÉRIMENTALE

### Isolation de la protéine

La protéine SEQ ID NO : 2 est obtenue à partir du plasma humain selon le procédé de Gan et *al.* (1991) suivant :

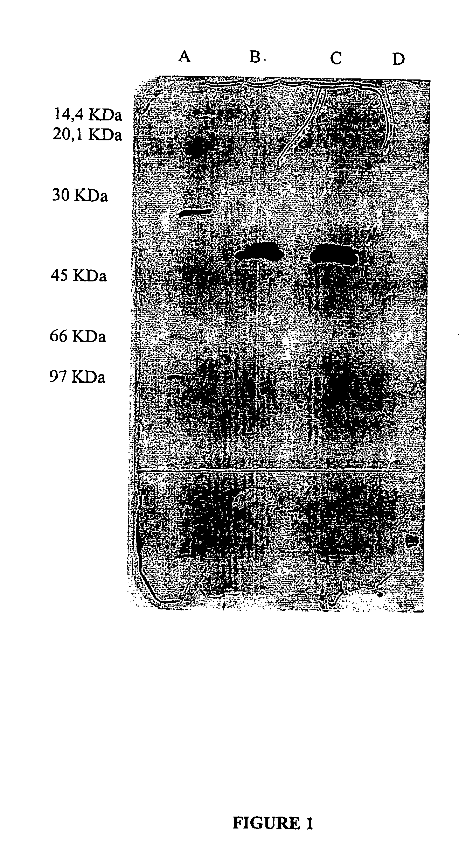

La protéine SEQ ID NO : 2 est purifiée à partir de poches de plasma congelé (∼200 ml) fournies par l'Etablissement de Transfusion Sanguine de Lyon-Beynost. Le caillot de fibrine, formé par l'ajout de 1 M (1% v/v) de CaCl₂ au plasma est séparé du sérum par filtration. Le sérum est alors mélangé à 400 ml de Gel d'affinité (Cibacron 3GA-Agarose, C-1535, Sigma) équilibré avec un tampon A (Tris/HCl 50 mM, CaCl₂ 1mM, NaCl 4M, pH 8). Dans ces conditions, principalement les HDL ("high density lipoprotein" : lipoprotéines de haute densité) sont adsorbées. Après 6 à 8 heures d'incubation, les protéines non adsorbées sur le gel sont éliminées par filtration sur fritté de porosité n°2. Ce lavage s'effectue jusqu'à ce que l'on ne détecte plus de protéine dans l'éluat (absorption UV à 280 nm). Le gel est ensuite équilibré avec un tampon B (Tris/HCl 50 mM, CaCl₂ 1mM, pH 8) puis placé en colonne XK 50/30 (Pharmacia). L'élution est réalisée en rajoutant 1g/l de déoxycholate de sodium et 0,1% de triton X-100 au tampon B. Les fractions montrant une activité arylestérase sont injectées sur 50 ml d'un gel échangeur d'anions (DEAE Sepharose Fast Flow, Pharmacia) disposé en colonne XK 26/70 (Pharmacia) et équilibré avec le tampon B et 0,05% de triton X-100. L'élution se fait par gradient de NaCl. Un premier palier est réalisé à 87,5 mM de NaCl afin d'éliminer l'apo A-I, une protéine liée à la paraoxonase, et la majorité des protéines contaminantes. La paraoxonase humaine (PON1) est environ éluée à la concentration de 140 mM de NaCl. Toutes les fractions conservées montrent une activité paraoxonase et arylestérase, ces activités étant vérifiées selon les tests mentionnés plus loin. Les fractions éluées ne sont pas regroupées. Les gels SDS-PAGE des fractions obtenues montrent des bandes comprises entre 38 kDa et 45 kDa (voir Figure 1). Chaque purification n'apporte pas toujours la même distribution de masse apparente. Cette légère hétérogénéité peut s'expliquer par la présence de 2 chaînes glycosylées sur la PON1.

En plus de la PON1 dans ces lots une autre protéine a été isolée par cristallisation, en substituant le triton par le C12-maltoside et en utilisant le sulfate d'ammonium comme agent précipitant. Les cristaux obtenus sont ceux d'une protéine inconnue caractérisée par radiocristallographie et correspondant à la séquence SEQ ID NO : 2 de l'invention. La cristallisation est actuellement le seul procédé existant pour purifier cette protéine.

L'activité paraoxonase est mesurée dans un tampon Glycine 50 mM/NaOH, CaCl₂ 1 mM, en présence de 1 M NaCl, pH 10,5 et est déterminée au moyen d'un spectrophotomètre à double faisceau (Shimadzu UV 160A) thermostaté à 25°C. La vitesse d'hydrolyse est déterminée d'après la variation d'absorbance à 412 nm, correspondant à la formation de p-nitrophénol libéré par l'hydrolyse de paraoxon, en fonction du temps, ε = 18290 M⁻¹cm⁻¹ (Smolen, 1991).

L'activité arylestérase est mesurée dans un tampon tris 50mM/HCl, CaCl₂ 1mM, pH 8 et est déterminée au moyen d'un spectrophotomètre à double faisceau (Shimadzu UV 160A) thermostaté à 25°C. La vitesse d'hydrolyse est déterminée d'après la variation d'absorbance à 270 nm, correspondant à la formation de phénol libéré par l'hydrolyse de phényl acétate, en fonction du temps, ε = 1310 M⁻¹cm⁻¹ (Smolen, 1991).

### Structure

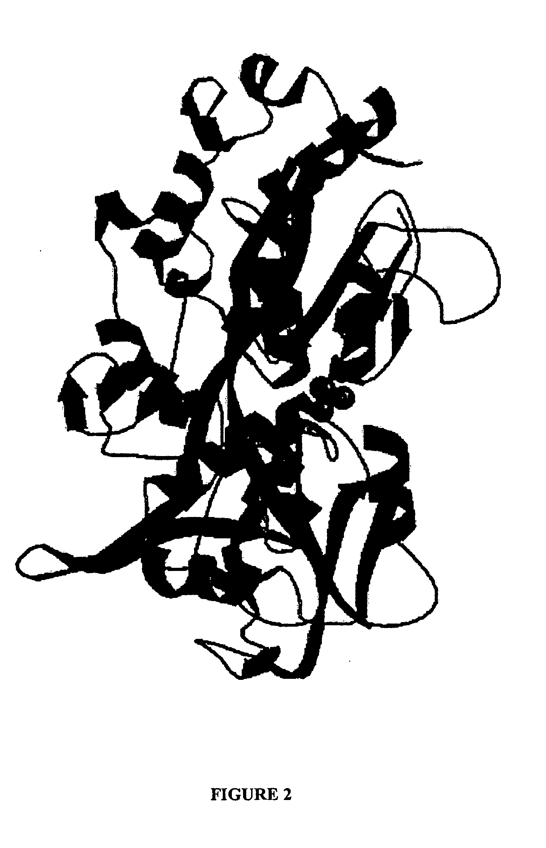

La structure de la protéine cristallisée SEQ ID NO : 2 a été obtenue par cristallographie des rayons X. La structure à 1,9 Å de résolution a été obtenue par la méthode SIRAS (Single Isomorphous Replacement and Anomalous Scattering)(Figure 2).

Les données de diffraction des rayons X ont été collectées sur la ligne BM30 de l'ESRF (Grenoble).

Un dérivé de sel d'atome lourd a été obtenu en trempant un cristal dans une solution contenant des sels d'uranium.

Les images ont été intégrées, mises à l'échelle et combinées avec les programmes XDS2000 (Kabsch, 1993) et la suite CCP4 (COLLABORATIVE COMPUTATIONAL PROJECT, NUMBER 4. 1994. "The CCP4 Suite: Programs for Protein Crystallography". Acta Cryst. D50, 760-763).

Les programmes CNS (BRUNGER, 1998) et SnB (Weeks, 1999) ont été utilisés pour localiser les atomes d'uranium. Le programme SHARP (Copyright © 2001-2002 the Buster Development Group) a été utilisé pour obtenir les phases par la technique SIRAS.

372 acides aminés ont été construits automatiquement dans la carte de densité électronique par le programme ARP/wARP (Perrakis, 1997). Ce premier modèle a ensuite été affiné par le programme CNS.

En raison de la très bonne qualité des cartes de densité électronique, la séquence primaire de la protéine a pu être assignée avec 80% de fiabilité. Une molécule de phosphate a aussi pu être localisée.

La structure obtenue ne correspond pas du tout à la paraoxonase humaine. Le séquençage obtenu en identifiant les acides aminés à partir de la densité électronique indique que ni cette protéine humaine ni son gène n'ont été décrits auparavant. Il s'agit donc d'une nouvelle protéine.

La structure de la protéine de l'invention montre une très forte homologie avec la protéine de fixation du phosphate ("phosphate binding") d*'Escherichia coli.* Cette protéine chez cette bactérie sert à transporter le phosphate à travers le périplasme. On la retrouve chez beaucoup de procaryotes mais chez aucun eucaryote.

La densité électronique a aussi montré qu'une molécule de phosphate était fixée à la nouvelle protéine de l'invention, de la même façon que dans celle d*'Escherichia coli.*

Ainsi, on peut conclure que la protéine de l'invention caractérisée à partir du plasma humain présente une très forte homologie avec la protéine bactérienne et qu'elle est capable de fixer le phosphate et de le transporter.

### Séquençage

### Digestion dans le gel

Le mélange paraoxonase-HPBP a été séparé par gel électrophorétique avec SDS-PAGE (sans chauffage). Plusieurs bandes correspondant à HPBP aux alentour de 70 kDa ont été découpées.

La digestion de la protéine contenue dans ces bandes a été effectuée grâce au système automatique de digestion, MassPrep Sation (Waters Manchester, G.B.). Les bandes de gel ont été lavées deux fois avec 50 µl d'une solution à 25 mM de carbonate d'ammonium hydrogéné (NH₄HCO₃) et 50 µl d'acétonitrile. Les cystéines ont été réduites avec 50 µl d'une solution à 10mM de dithiothréitol à 57°C et acylé avec 50 µl de iodocacétamide à 55 mM. Après déshydratation avec l'acétonitrile, la protéine a été digérée enzymatiquement avec 10 µl de trypsine porcine modifiée à 12,5 ng/µl (Promega, Madisson, WI, U.S.A) ou bien avec lys-C de Lysobacter enzymogenes (Roche Applied Science, Penzberg, Germany) dans 25 mM de NH₄HCO₃. La digestion s'est opérée une nuit complète à température ambiante. Les peptides clivés ont été extraits avec une solution à 60% d'acétonitrile et 5% d'acide formique.

### Analyse par spectrométrie de masse

### MALDI-MS et MALDI-MS/MS

Les mesures de masse par MALDI-TOF on été effectuées sur un Ultraflex™ TOF/TOF (Bruker, Daltonik GmbH, Brème, Allemagne). Cet instrument a été utilisé avec un potentiel d'accélération maximum de 25 KV dans le mode reflectron. L'échantillon a été préparé avec la préparation standard de la goutte asséchée sur la cible en acier inoxydable en utilisant comme matrice l'acide α-cyano-4-hydroxycinnamique.

La calibration externe du spectre MALDI-MS a été effectuée en utilisant seulement les pics des charges mono isotopiques d'une solution connue de peptides (bradykinine 1-7 (m/z=757,400), angiotensine humaine II (m/z=1046,542), angiotensine humaine I (m/z=1296,685), substance P (m/z=1347,735), bombesine (m/z=1619,822), renine (m/z=1758,933), ACTH 1-17 (m/z=2093,087) et ACTH 18-39 (m/z=2465,199)). Les masses des peptides mono isotopiques ont été automatiquement annotées grâce au programme Flexanalysis 2.0.

Les spectres MS/MS ont été obtenus par l'analyse des ions métastables obtenus par "Laser-Induced Decomposition" (LID) d'un précurseur ioniques sectionné, sans collision additionnelle en phase gazeuse. Le précurseur ionique a été accéléré à 8kV et a été sélectionné grâce à une trappe à ions à sélection temporelle. Les fragments ont été par la suite accélérés à 19 kV dans la cellule LIFT et leurs masses mesurées après leurs passages sur le réflecteur ionique.

Le séquençage *de novo* de chacun de ces spectres MS/MS a été effectué avec le programme Full DeNovo Sequencing program (Biotools, Bruker Daltonik GmbH, Brème, Allemagne).

### NanoLC-MS/MS

L'analyse NanoLC-MS/MS a été effectuée en utilisant un CapLC (Waters, Manchester, G.B.) couplé à un spectromètre de masse "temps de vol" accéléré par un quadripôle hybride orthogonal Q-TOF II ((Micromass, Manchester, G.B.). La séparation par chromatographie en phase inverse à été effectuée avec des capillaires (Pepmap C18, 75 µm i.d., 15 cm de long, LC Packings) sous un flux à 200 nL/min, maintenu constant grâce à une pré-colonne de partage. La calibration a été effectuée en utilisant 2pmol/µl de GFP.

L'acquisition des données de masse a été pilotée par le programme MassLynx (Micromass, Manchester, G.B.) qui bascule automatiquement entre le mode MS et le mode MS/MS.

Les spectres MS/MS générés ont été individuellement séquencés *de novo* afin d'obtenir la séquence partielle ou complète. Ces interprétations ont été réalisées en utilisant le programme PepSeq (MassLynx, Micromass) et le programme PEAKS Studio (Bioinformatics Solutions, Waterloo, Canada) qui sont capables de traiter complètement un fichier .pkl avec un séquençage *de novo* automatique sur chaque spectre MS/MS.

### Fixation du phosphate

La fixation du phosphate par la protéine de l'invention SEQ ID NO : 2 a été mise en évidence selon le test suivant :
On dépose 200 µl de la protéine de l'invention SEQ ID NO : 2 (colonnes A-F de la Figure 3), ou du lysozyme 1 mg/ml (colonne G) ou de la βlacto-globuline sur nitrocellulose (dot blot par aspiration).
L'ensemble est incubé pendant 2 h 30 dans un mélange comprenant : tris 50 mM ; pH 8,0 ; ³²P (10 mCi/ml) 2 mM.
On effectue ensuite un rinçage 2 fois pendant 1 minute avec du tris 50 mM à pH 8,0, puis on expose l'ensemble à température ambiante pendant 45 minutes.
On constate alors (voir Figure 3) que la protéine de l'invention a fixé le phosphate radioactif (colonnes A à F), alors que les témoins tests ne l'ont pas fixée (colonnes G et H).

### Rôle et utilisation de la protéine SEQ ID NO : 2

Pour doser la concentration de cette protéine dans le plasma les méthodes utilisables sont :
- les methodes électrophorétiques,
- la purification de la protéine,
- la quantification de son activité,
- l'immunodosage de la protéine en utilisant des anticorps polyclonaux/monoclonaux dirigé contre la protéine.

### Association avec la paraoxonase

### Electrophorèse bidimensionnelle

Les protéines purifiées (40 µg) comme dans le protocole décrit précédemment sont mélangées à 100 µL d'une solution contenant 9,8 M d'urée, 4% (v/v) triton X100, 2 mM tributyl phosphine, 0,2 % (v/v) d'ampholine 3-10 (Bio-Lytes 3 -10 ; Bio-Rad), et 0,001 % (m/v) de bleu de bromophénol. Des bandelettes (IPG-Strips ; Bio-Rad) de gel de polyacrylamide (T : 4 % ; C : 3 %) prêtes à l'emploi sont utilisées. Des ampholines ont été fixées de manière covalente au polyacrylamide de sorte d'avoir un gradient linéaire de pH pré-établi. Le gradient de pH utilisé est entre 3,0 et 10,0.

### 1. Isoélectrofocation (IEF)

Les bandelettes sont placées en contact avec les échantillons protéiques dans l'appareil Protean IEF Cell (Bio-Rad) et réhydratées activement (50 V constant) pendant 15 heures à 20°C. L'isoélectrofocalisation est ensuite réalisée en 3 étapes à 20°C. Premièrement un faible voltage de 250 V est appliqué pendant 15 minutes ; deuxièmement, une montée en gradient de 250 à 4000 V (ampérage limité par bandelette à 50 µA) est programmée sur 2 h. Troisièmement, le voltage est maintenu constant à 4000 V pendant 4 heures. Après migration, les bandelettes sont conservées à -20°C.

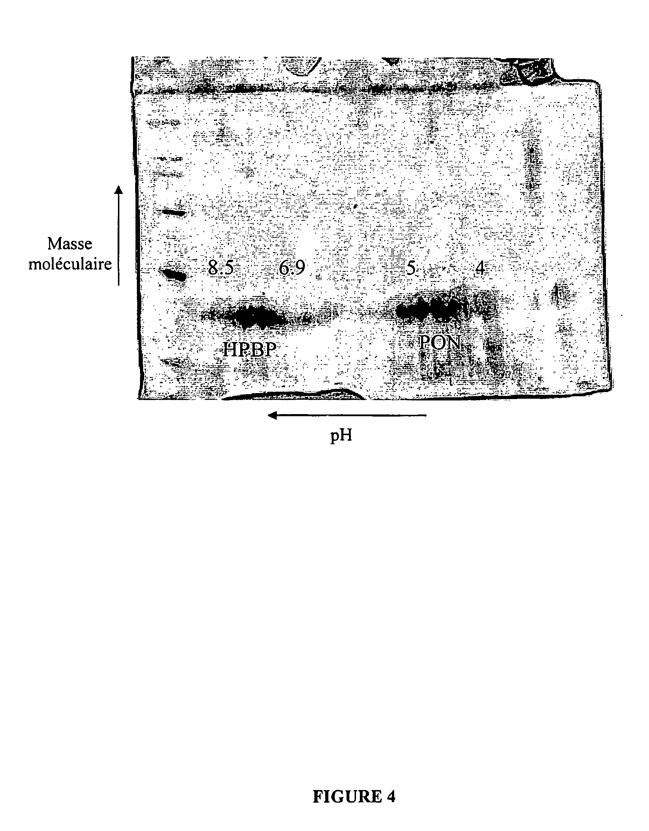

D'après le protocole de purification précédent, la protéine HPBP de l'invention est co-purifiée avec la paraoxonase humaine (PON)(Fokine et al., 2003). En faisant un gel bidimensionnel avec le protocole ci-dessus, 2 spots ont été identifiés par séquençage N-terminal comme étant respectivement la protéine de l'invention HPBP et la paraoxonase humaine (voir Figure 4). Les deux protéines ont approximativement la même masse moléculaire (environ 40 kDa) et des points isoélectriques distincts, 6.9-8.5 pour HPBP et 4-5 pour la PON1. En tenant compte du fait qu'il a fallu utiliser des conditions drastiques pour réussir à séparer sur gel les 2 protéines (9M d'urée et 4% de triton)et que les 2 protéines qui ont des points isoélectriques très différents restent co-purifiées après le passage dans une colonne échangeuse d'anion (DEAE sepharose), on conclut qu'elles sont associées en formant un complexe.

### RÉFÉRENCES BIBLIOGRAPHIQUES

- Amann K., Tornig J., Kugel B., Gross M.L., Tyralla K., El-Shakmak A., Szabo A., Ritz E. (2003) Kidney Int. 63(4): 1296-1301 ;
- Blazheevich N.V., Spirichev V.B., Pozdniakov A.L. (1975) Kardiologiia. 15(6): 67-71 ;
- Brunger A.T., Adams P.D., Clore G.M., Delano W.L., Gros P., Grosse-Kunstleve R.W., Jiang J.-S., Kuszewski J., Nilges N., Pannu N.S., Read R.J., Rice L.M., Simonson T. et Warren G.L. (1998) Acta Cryst. D54: 905-921 ;
- Dorozhkin S.V., Epple M. (2002) Angew Chem Int Ed Engl. 41(17): 3130-46 ;
- Engvall E., Jonsson K., Perlmann P. (1971) Biochim Biophys Acta. (1971) 251: 427-34 ;
- Fokine A., Morales R., Contreras-Martel C., Carpentier P., Renault F., Rochu D., Chabriere E. (2003) Acta Crystallogr D. 59, 2083-7 ;
- Gan, K.N., Smolen, A., Eckerson, H.W. & La Du, B.N. (1991). Drug Metab Dispos. 19, 100-106 ;
- Hassett, C., Richter, R.J., Humbert, R., Chapline, C., Crabb, J.W., Omiecinski, C.J. et Furlong, C.E. (1991) Biochemistry 30(42), 10141-10149 ;
- Kabsch W. (1993) J. Appl. Cryst. 26: 795-800 ;
- Perrakis, A., Sixma, T. K., Wilson, K.S., et Lamzin, V. S. (1997) Acta Cryst. D53: 448-455 ;
- Primo-Parmo, S.L., Sorenson, R.C., Teiber, J. et La Du, B.N. (1996) Genomics 33(3), 498-507 ;
- Ramsdell R. (1999) Anna J. 26(3): 346-7 ;
- Reddy, S.T., Wadleigh, D.J., Grijalva, V., Ng, C., Hama, S., Gangopadhyay, A., Khorsan, R., Shih, D., Lusis, A.J., Navab, M. et Fogelman,A.M. (2001) Arterioscler Thromb Vasc Biol 21(4):542-7 ;
- Rodrigo, L., Gil, F., Hemandez, A.F., Marina, A., Vazquez, J. et Pla, A. (1997) Biochem. J. 321, 595-601 ;
- Smolen A, Eckerson HW, Gan KN, Hailat N, La Du BN. (1991) Drug Metab Dispos., 19: 107-112 *;*
- Sorenson, R.C., Primo-Parmo, S.L., Kuo, C.L., Adkins, S., Lockridge, O. et La Du, B.N. (1995) Proc. Natl. Acad. Sci. U.S.A. 92(16), 7187-7191 ;
- Weeks, C.M. & Miller, R. (1999) J. Appl. Cryst. 32,120-124.

## Revendications

1. Protéine **caractérisée en ce qu'**elle comprend ou est constituée par :
- la séquence SEQ ID NO : 1,
- ou toute séquence homologue de la séquence SEQ ID NO : 1, ayant une homologie d'au moins 80% avec la séquence SEQ ID NO : 1, sous réserve que ladite séquence homologue se lie au phosphate.

2. Protéine selon la revendication 1, **caractérisée en ce qu'**elle comprend ou est constituée par :
- la séquence SEQ ID NO : 2 ou SEQ ID NO : 3,
- ou toute séquence homologue de la séquence SEQ ID NO : 2 ou SEQ ID-NO : 3, ayant une homologie d'au moins 80% avec la séquence SEQ ID NO : 2 ou SEQ ID NO : 3, sous réserve que ladite séquence homologue se lie au phosphate.

3. Séquence nucléotidique codant pour une protéine telle que définie dans la revendication 1 ou 2.

4. Vecteur recombinant, plasmide, cosmide, phage ou ADN de virus, contenant une séquence nucléotidique selon la revendication 3.

5. Vecteur recombinant selon la revendication 4, contenant les éléments nécessaires à l'expression dans une cellule hôte des polypeptides codés par une séquence nucléotidique selon la revendication 3, insérés dans ledit vecteur.

6. Cellule hôte isolée, choisie parmi les bactéries, les levures, les cellules de champignons, les cellules de plantes ou les cellules de mammifères, ladite cellule hôte étant transformée à l'aide d'un vecteur recombinant selon l'une des revendications 4 ou 5.

7. Composition pharmaceutique comprenant à titre de substance active une protéine selon la revendication 1 ou 2, en association avec un véhicule pharmaceutiquement acceptable.

8. Composition pharmaceutique selon la revendication 7, comprenant à titre de substance active une protéine représentée par la séquence SEQ ID NO : 2 ou SEQ ID NO:3.

9. Composition pharmaceutique selon la revendication 8, dans laquelle la protéine telle que définie dans la revendication 1 ou 2, est en association avec un variant de la protéine paraoxonase, de SEQ ID NO : 4, SEQ ID NO : 5, SEQ ID NO : 6, SEQ ID NO : 7, SEQ ID NO : 8, SEQ ID NO : 9, SEQ ID NO : 10 ou SEQ ID NO : 11.

10. Utilisation d'une protéine selon la revendication 1 ou 2, pour la préparation d'un médicament destiné à la prévention ou au traitement de l'arthrite ou de maladies liées à une hyperphosphatémie, telles que les maladies cardiovasculaires, ou, en association avec un variant de la protéine paraoxonase de SEQ ID NO : 4, SEQ ID NO : 5, SEQ ID NO : 6, SEQ ID NO : 7, SEQ ID NO : 8, SEQ ID NO : 9, SEQ ID NO : 10 ou SEQ ID NO : 11, dans le cadre de la prophylaxie ou du traitement des intoxications provoquées par des insecticides ou des agents neurotoxiques tels que le soman, le VX, le sarin ou le tabun, ou dans le cadre du traitement de l'athérosclérose.

11. Produit de combinaison comprenant au moins une protéine selon la revendication 1 ou 2, et au moins un variant de la protéine paraoxonase de SEQ ID NO : 4, SEQ ID NO : 5, SEQ ID NO : 6, SEQ ID NO : 7, SEQ ID NO : 8, SEQ ID NO : 9, SEQ ID NO : 10 ou SEQ ID NO : 11, pour une utilisation simultanée, séparée ou étalée dans le temps destiné à la prophylaxie ou au traitement des intoxications provoquées par des insecticides ou des agents neurotoxiques tels que le soman, le VX, le sarin ou le tabun.

12. Méthode de dosage de la protéine telle que définie dans la revendication 1
ou 2, ladite méthode étant choisie parmi
- les méthodes électrophorétiques,
- la purification de ladite protéine,
- la quantification de son activité, et
- l'immunodosage de ladite protéine en utilisant des anticorps polyclonaux/monoclonaux dirigé contre ladite protéine.

13. Application de la méthode de dosage selon la revendication 12
- au diagnostic *in vitro* de maladies liées à une hyperphosphatémie notamment lorsque la quantité de protéine selon la revendication 1 ou 2, dosée selon la méthode de la revendication 12, est inférieure à la quantité de cette protéine normalement présente dans le sang d'un individu sain, ou
- au diagnostic *in vitro* de maladies liées à une hypophosphatémie notamment lorsque la quantité de protéine selon la revendication 1 ou 2, dosée selon la méthode de la revendication 12, est supérieure à la quantité de cette protéine normalement présente dans le sang d'un individu sain, ou
- au diagnostic *in vitro* d'une prédisposition d'un individu à de telles pathologies.

14. Application selon la revendication 13 au diagnostic *in vitro* de maladies liées à une hyperphosphatémie telles que les maladies cardiovasculaires liées à la formation de plaques d'athéromes, ou au diagnostic *in vitro* d'une prédisposition d'un individu au développement d'une des maladies susmentionnées.

15. Application selon la revendication 13 au diagnostic *in vitro* de maladies liées à une hypophosphatémie, ou au diagnostic *in vitro* d'une prédisposition d'un individu au développement de ces maladies.

## Claims

1. Protein **characterized in that** it comprises or is constituted by:
- the sequence SEQ ID NO: 1,
- or any sequence homologous to the sequence SEQ ID NO: 1, having a homology of at least approximately 80% with the sequence SEQ ID NO: 1, providing that said homologous sequence binds to phosphate.

2. Protein of claim 1, **characterized in that** it comprises or is constituted by:
- the sequence SEQ ID NO: 2 or SEQ ID NO: 3,
- or any sequence homologous to the sequence SEQ ID NO: 2 or SEQ ID NO: 3, having a homology of at least approximately 80% with the sequence SEQ ID NO: 2 or SEQ ID NO: 3, providing that said homologous sequence binds to phosphate.

3. Nucleotide sequence encoding a protein as defined in claim 1 or 2.

4. Recombinant vector, in particular plasmid, cosmid, phage or virus DNA, containing a nucleotide sequence according to claim 3.

5. Recombinant vector according to claim 4, containing the elements necessary for the expression in a host cell of the polypeptides encoded by a nucleotide sequence according to claim 3, inserted into said vector.

6. Isolated host cell, chosen in particular from bacteria, yeasts, fungi cells, plant cells or mammal cells, said host cell being transformed using a recombinant vector according to claim 4 or 5.

7. Pharmaceutical composition comprising as active ingredient a protein according to claim 1 or 2, in combination with a pharmaceutically acceptable vehicle.

8. Pharmaceutical composition according to claim 7, comprising as active ingredient a protein represented by the sequence SEQ ID NO: 2 or SEQ ID NO: 3.

9. Pharmaceutical composition according to claim 8, in which the protein such as defined in claim 1 or 2 is in combination with a variant of the paraoxonase protein ofSEQ ID NO: 4, SEQ ID NO: 5, SEQ ID NO: 6, SEQ ID NO: 7, SEQ ID NO: 8, SEQ ID NO: 9, SEQ ID NO: 10 or SEQ ID NO: 11.

10. Use of a protein according to claim 1 or 2, for the preparation of a drug intended for the prevention or treatment of arthritis or diseases linked to hyperphosphataemia, such as cardiovascular diseases,
or in association with a variant of the paraoxonase protein of SEQ ID NO: 4, SEQ ID NO: 5, SEQ ID NO: 6, SEQ ID NO: 7, SEQ ID NO: 8, SEQ ID NO: 9, SEQ ID NO: 10 or SEQ ID NO: 11 intended for the prophylaxis or treatment of intoxications caused by insecticides or nerve agents such as soman, VX, sarin or tabun, or intended for the treatment of artherosclerosis.

11. A combination product comprising at least one protein according to claim 1, in particular SEQ ID NO: 2 or SEQ ID NO: 3, and at least one variant of the paraoxonase protein, in particular of SEQ ID NO: 4, SEQ ID NO: 5, SEQ ID NO: 6, SEQ ID NO: 7, SEQ ID NO: 8, SEQ ID NO: 9, SEQ ID NO: 10 or SEQ ID NO: 11, for simultaneous or separate use, or use spread over time, intended for the prophylaxis or treatment of intoxications caused by insecticides or nerve agents such as soman, VX, sarin or tabun.

12. Method of dosage of the protein according to claim 1 or 2, said method being chosen among :
- electrophoretic methods,
- purification of said protein,
- quantification of its activity,
immunodosage of said protein by using monoclonal/polyclonal antibodies directed against said protein,

13. Application of the method of dosage according to claim 12,
- for *the in vitro* diagnosis of diseases linked to hyperphosphataemia in particular when the quantity of protein according to claim 1 or 2, assayed according to the method of claim 12, is less than the quantity of this protein normally present in the blood of a healthy individual, or
- for the in vitro diagnosis of diseases linked to hypophosphataemia in particular when the quantity of protein according to claim 1 or 2, assayed according to the method of claim 12, is greater than the quantity of this protein normally present in the blood of a healthy individual, or
- for the *in vitro* diagnosis of an individual's predisposition to such pathologies.

14. Application of the method of dosage according to claim 13, for the *in vitro* diagnosis of diseases linked to hyperphosphataemia such as cardiovascular diseases linked to the formation of atheroma plaques, or for *the in vitro* diagnosis of an individual's predisposition to develop one of the above-mentioned diseases.

15. Application of the method of dosage according to claim 13, for the *in vitro* diagnosis of diseases linked to hypophosphataemia, or for the *in vitro* diagnosis of an individual's predisposition to develop one of the above-mentioned diseases.

## Patentansprüche

1. Protein, **dadurch gekennzeichnet, dass** es umfasst oder besteht aus:
- der Sequenz SEQ ID NR: 1,
- oder jeder homologen Sequenz der Sequenz SEQ ID NR: 1, die eine Homologie von mindestens 80 % mit der Sequenz SEQ ID NR: 1 aufweist, unter der Bedingung, dass die homologe Sequenz an das Phosphat bindet.

2. Protein nach Anspruch 1, **dadurch gekennzeichnet, dass** es umfasst oder besteht aus:
- der Sequenz SEQ ID NR: 2 oder SEQ ID NR: 3,
- oder jeder homologen Sequenz der Sequenz SEQ ID NR: 2 oder SEQ ID NR: 3, die eine Homologie von mindestens 80 % mit der Sequenz SEQ ID NR: 2 oder SEQ ID NR: 3 aufweist, unter der Bedingung, dass die homologe Sequenz an das Phosphat bindet,

3. Nukleotidsequenz, die für ein Protein kodiert, wie in Anspruch 1 oder 2 definiert.

4. Rekombinanter Vektor, insbesondere Plasmid, Cosmid, Phage oder Virus-DNA, der eine Nukleotidsequenz nach Anspruch 3 enthält.

5. Rekombinanter Vektor nach Anspruch 4, der die Elemente enthält, die für die Expression in einer Wirtszelle der Polypeptide, die von einer Nukleotidsequenz nach Anspruch 3 kodiert werden, eingefügt in den Vektor, notwendig sind.

6. Isolierte Wirtszelle, insbesondere ausgewählt aus Bakterien, Hefen, Pilzzellen, Pflanzenzellen oder Säugerzellen, wobei die Wirtszelle mit Hilfe eines rekombinanten Vektors nach einem der Ansprüche 4 oder 5 transformiert wird.

7. Pharmazeutische Zusammensetzung, die als Wirkstoff ein Protein nach Anspruch 1 oder 2, in Assoziation mit einem pharmazeutisch annehmbaren Vehikel umfasst.

8. Pharmazeutische Zusammensetzung nach Anspruch 7, die als Wirkstoff ein Protein umfasst, das durch die Sequenz SEQ ID NR: 2 oder SEQ ID NR: 3 dargestellt wird.

9. Pharmazeutische Zusammensetzung nach Anspruch 8, worin das Protein, wie in Anspruch 1 oder 2 definiert, mit einer Variante des Proteins Paraoxonase, von SEQ ID NR: 4, SEQ ID NR: 5, SEQ ID NR: 6, SEQ ID NR: 7, SEQ ID NR: 8, SEQ ID NR: 9, SEQ ID NR: 10 oder SEQ ID NR: 11 assoziiert ist.

10. Verwendung eines Proteins nach Anspruch 1 oder 2 zur Herstellung eines Medikaments zur Vorbeugung oder Behandlung von Arthritis oder von Krankheiten, die mit einer Hyperphosphatämie verbunden sind, wie kardiovasukäre Krankheiten, oder, in Assoziation mit einer Variante des Proteins Paraoxonase von SEQ ID NR: 4, SEQ ID NR: 5, SEQ ID NR: 6, SEQ ID NR: 7, SEQ ID NR: 8, SEQ ID NR: 9, SEQ ID NR: 10 oder SEQ ID NR: 11, im Rahmen der Prophylaxe oder der Behandlung von Vergiftungen, die von Insektiziden oder neurotoxischen Mitteln, wie etwa Soman, VX, Sarin oder Tabun hervorgerufen werden, oder im Rahmen der Behandlung von Atherosklerose.

11. Kombinationsprodukt, das mindestens ein Protein nach Anspruch 1 oder 2 und mindestens eine Variante des Proteins Paraoxonase von SEQ ID NR: 4, SEQ ID NR: 5, SEQ ID NR: 6, SEQ ID NR: 7, SEQ ID NR: 8, SEQ ID NR: 9, SEQ ID NR: 10 oder SEQ ID NR: 11 umfasst, zur gleichzeitigen, getrennten oder zeitlich gestaffelten Verwendung zur Prophylaxe oder zur Behandlung von Vergiftungen, die von Insektiziden oder neurotoxischen Mitteln, wie Soman, VX, Sarin oder Tabun hervorgerufen werden.

12. Verfahren zur Bestimmung des Proteins, wie in Anspruch 1 oder 2 definiert, wobei das Verfahren ausgewählt ist aus
- den elektrophoretischen Verfahren,
- der Reinigung des Proteins,
- der Quantifikation dessen Aktivität, und
- der Immunbestimmung des Proteins unter Verwendung von polyklonalen/monoklonalen Antikörpern, die gegen das Protein gerichtet sind.

13. Anwendung des Verfahrens zur Bestimmung nach Anspruch 12
- zur In-vitro-Diagnose von Krankheiten, die mit einer Hyperphosphatämie verbunden sind, insbesondere wenn die Menge an Protein nach Anspruch 1 oder 2, die nach dem Verfahren aus Anspruch 12 bestimmt wurde, kleiner ist als die Menge dieses Proteins, die normalerweise im Blut eines gesunden Individuums vorhanden ist, oder
- zur In-vitro-Diagnose von Krankheiten, die mit einer Hypophosphatämie verbunden sind, insbesondere wenn die Menge an Protein nach Anspruch 1 oder 2, die nach dem Verfahren aus Anspruch 12 bestimmt wurde, größer ist als die Menge dieses Proteins, die normalerweise im Blut eines gesunden Individuums vorhanden ist, oder
- zur In-vitro-Diagnose einer Prädisposition eines Individuums für solche Krankheitserscheinungen.

14. Anwendung nach Anspruch 13 zur In-vitro-Diagnose von Krankheiten, die mit einer Hyperphosphatämie verbunden sind, wie die kardiovaskulären Krankheiten, die mit einer Bildung von Atheromplaques verbunden sind, oder zur In-vitro-Diagnose einer Prädisposition eines Individuums zur Entwicklung einer der oben genannten Krankheiten.

15. Anwendung nach Anspruch 13 zur In-vitro-Diagnose von Krankheiten, die mit einer Hypophosphatämie verbunden sind, oder zur In-vitro-Diagnose einer Prädisposition eines Individuums zur Entwicklung dieser Krankheiten.
